# EUROPEAN PATENT SPECIFICATION

(11) **EP 1 119 615 B1**
(45) Date of publication and mention of the grant of the patent: **27.09.2006**
(21) Application number: 99950296.6
(22) Date of filing: 12.10.1999
(51) Int. Cl.: C12N 15/10, C12N 15/11, C12N 15/85, C12N 5/10, A61K 31/711, A61K 48/00

(54) **PRODUCTION OF SSDNA INSIDE A CELL**
HERSTELLUNG VON SSDNA INNERHALB DER ZELLE
PRODUCTION D'ADN SIMPLE BRIN A L'INTERIEUR D'UNE CELLULE

(30) Priority: 09.10.1998 US 169793; 16.09.1999 US 397782; 04.10.1999 US 411568
(43) Date of publication of application: 01.08.2001
(73) Proprietor: CytoGenix, Inc., Houston, TX 77042 (US)
(72) Inventor: CONRAD, Charles, A., Spring, Texas 77379-6800 (US); Yin Chen, Pearland TX 77584 (US)
(74) Representative: Weihs, Bruno Konrad
(86) International application number: PCT/US1999/023936
(87) International publication number: WO 2000/022114

(56) References cited:
- EP-A- 0 530 112
- EP-A- 0 532 380
- EP-A- 0 562 206
- WO-A-94/01550
- WO-A-94/13689
- WO-A-94/20639
- WO-A-94/23026
- WO-A-95/35369
- O. MIROCHNITCHENKO ET AL.: "Production of single-stranded DNA in mammalian cells by means of a bacterial retron" J. BIOL. CHEM., vol. 269, no. 4, 28 January 1994 (1994-01-28), pages 2380-2383, XP002132577 AM. SOC. BIOCHEM. MOL.BIOL.,INC.,BALTIMORE,US cited in the application
- J.-R. MAO ET AL.: "Gene regulation by antisense DNA produced in vivo" J. BIOL. CHEM., vol. 270, no. 34, 25 August 1995 (1995-08-25), pages 19684-19687, XP002132578 AM. SOC. BIOCHEM. MOL.BIOL.,INC.,BALTIMORE,US cited in the application
- OHSHIMA A ET AL: "IN-VIVO DUPLICATION OF GENETIC ELEMENTS BY THE FORMATION OF STEM-LOOP DNA WITHOUT AN RNA INTERMEDIATE" PROCEEDINGS OF THE NATIONAL ACADEMY OF SCIENCES OF THE UNITED STATES OF AMERICA 1992, vol. 89, no. 3, 1992, pages 1016-1020, XP002132579 ISSN: 0027-8424 cited in the application

### SEQUENCE LISTING

<110> INGENE, INC.
<120> PRODUCTION OF ssDNA *IN VIVO*
<130> INGA,004/PCT
<140> PCT/US99/23936
   <141> 1999-10-12
<160> 45
<170> PatentIn Ver. 2.0
<210> 1
   <211> 24
   <212> DNA
   <213> Artificial Sequence
<220>
   <223> Description of Artificial Sequence: Synthetic oligonucleotide
<400> 1
   cttgtgcaca agctttgcag gtct 24
<210> 2
   <211> 32
   <212> DNA
   <213> Artificial Sequence
<220>
   <223> Description of Artificial Sequence: Synthetic oligonucleotide
<400> 2
   gggatcagga gctcagatca tgggaccaat gg 32
<210> 3
   <211> 30
   <212> DNA
   <213> Artificial Sequence
<220>
   <223> Description of Artificial Sequence: Synthetic oligonucleotide
<400> 3
   caattaagga aagctttgaa aaattatgtc 30
<210> 4
   <211> 129
   <212> DNA
   <213> Artificial Sequence
<220>
   <223> Description of Artificial Sequence: Synthetic oligonucleotide
<400> 4
   ctaggtcggc ggccgcgaag attggtgcgc acacacacaa cgcgcaccaa tcttcgcggc 60
   cgccgacccg tcagcggggg tctttcattt gggggctcgt ccgggatcgg gagacccctg 120
   cccagggcc                                                         129
<210> 5
   <211> 121
   <212> DNA
   <213> Artificial Sequence
<220>
   <223> Description of Artificial Sequence: Synthetic oligonucleotide
<400> 5
   ctgggcaggg gtctcccgat cccggacgag cccccaaatg aaagaccccc gctgacgggt 60
   cggcggccgc gaagattggt gcgcgttgtg tgtgtgcgca ccaatcttcg cggccgccga 120
   c                                                                 121
<210> 6
   <211> 18
   <212> DNA
   <213> Artificial Sequence
<220>
   <223> Description of Artificial Sequence: Synthetic oligonucleotide
<400> 6
   ctagcggcaa gcgtagct 18
<210> 7
   <211> 10
   <212> DNA
   <213> Artificial Sequence
<220>
   <223> Description of Artificial Sequence: Synthetic oligonucleotide
<400> 7
   acgcttgccg 10
<210> 8
   <211> 27
   <212> DNA
   <213> Artificial Sequence
<220>
   <223> Description of Artificial Sequence: Synthetic oligonucleotide
<400> 8
   taatggcccg ggcatagtcg ggtaggg 27
<210> 9
   <211> 43
   <212> DNA
   <213> Artificial Sequence
<220>
   <223> Description of Artificial Sequence: Synthetic oligonucleotide
<400> 9
   agctggatcc cccgctcccc accaccacca ccaccctgcc cct 43
<210> 10
   <211> 42
   <212> DNA
   <213> Artificial Sequence
<220>
   <223> Description of Artificial Sequence: Synthetic oligonucleotide
<400> 10
   agcaggggca gggtggtggt ggtggtgggg agcgggggat cc 42
<210> 11
   <211> 57
   <212> DNA
   <213> Artificial Sequence
<220>
   <223> Description of Artificial Sequence: Synthetic oligonucleotide
<400> 11
   ggccggaaga ttggggcgcc aaagagtaac tctcaaaggc acgcgcccca atcttcc 57
<210> 12
   <211> 57
   <212> DNA
   <213> Artificial Sequence
<220>
   <223> Description of Artificial Sequence: Synthetic oligonucleotide
<400> 12
   ggccggaaga ttggggcgcg tgcctttgag agttactctt tggcgcccca atcttcc 57
<210> 13
   <211> 92
   <212> DNA
   <213> Artificial Sequence
<220>
   <223> Description of Artificial Sequence: Synthetic oligonucleotide
<400> 13
   ggccggaaga ttggggcgtt agggttaggg ttagggttag ggttagggtt agggttaggg 60
   ttagggttag ggttagggcg ccccaatctt cc                                92
<210> 14
   <211> 92
   <212> DNA
   <213> Artificial Sequence
<220>
   <223> Description of Artificial Sequence: Synthetic oligonucleotide
<400> 14
   ggccggaaga ttggggcgcc ctaaccctaa ccctaaccct aaccctaacc ctaaccctaa 60
   ccctaaccct aaccctaacg ccccaatctt cc                                92
<210> 15
   <211> 111
   <212> DNA
   <213> Artificial Sequence
<220>
   <223> Description of Artificial Sequence: Synthetic oligonucleotide
<400> 15
   ctagtcggat caggccgctg cacaacaaca cacaacacag cggccgcatc cgatcagcgg 60
   gggtctttca tttgggggct cgtccggatc gggagacccc tgcccagcgc c          111
<210> 16
   <211> 103
   <212> DNA
   <213> Artificial Sequence
<220>
   <223> Description of Artificial Sequence: Synthetic oligonucleotide
<400> 16
   ctgggcaggg gtctcccgat ccggacgagc ccccaaatga aagacccccg ctgatcggat 60
   gcggccgctg tgttgtttgt tgttgtgcag cggccgcatc cga 103
<210> 17
   <211> 149
   <212> DNA
   <213> Artificial Sequence
<220>
   <223> Description of Artificial Sequence: Synthetic oligonucleotide
<400> 17
   agcttggtcg gcggccttga agagcggccg cactcacgat agagtgggag atgggcgcga 60
   gaaagtgcgg ccgctcttca aggccgccga ccttaattaa gtcagcgggg gatccttttt 120
   gggggctcgt ccgggatcgg gagacccct 149
<210> 18
   <211> 149
   <212> DNA
   <213> Artificial Sequence
<220>
   <223> Description of Artificial Sequence: Synthetic oligonucleotide
<400> 18
   ggccaggggt ctcccgatcc cggacgagcc cccaaaaagg atcccccgct gacttaatta 60
   aggtcggcgg ccttgaagag cggccgcact ttctcgcgcc catctcccac tctatcgtga 120
   gtgcggccgc tcttcaaggc cgccgacca 149
<210> 19
   <211> 30
   <212> DNA
   <213> Artificial Sequence
<220>
   <223> Description of Artificial Sequence: Synthetic oligonucleotide
<400> 19
   gatgtaagtc gttgtagcta gcctcccctg 30
<210> 20
   <211> 36
   <212> DNA
   <213> Artificial Sequence
<220>
   <223> Description of Artificial Sequence: Synthetic oligonucleotide
<400> 20
   gatccagggg aggctagcta caacgactta catcat 36
<210> 21
   <211> 35
   <212> DNA
   <213> Artificial Sequence
<220>
   <223> Description of Artificial Sequence: Synthetic oligonucleotide
<400> 21
   ggtgggcgcc tcgttgtagc tagcctcggt gtggg 35
<210> 22
   <211> 41
   <212> DNA
   <213> Artificial Sequence
<220>
   <223> Description of Artificial Sequence: Synthetic oligonucleotide
<400> 22
   gatccccaca ccgaggctag ctacaacgag gcgcccacca t 41
<210> 23
   <211> 34
   <212> DNA
   <213> Artificial Sequence
<220>
   <223> Description of Artificial Sequence: Synthetic oligonucleotide
<400> 23
   aatgcatgtc tcgttgtagc tagcccaggc ggga 34
<210> 24
   <211> 40
   <212> DNA
   <213> Artificial Sequence
<220>
   <223> Description of Artificial Sequence: Synthetic oligonucleotide
<400> 24
   gatctcccgc ctgggctagc tacaacgaga catgcattat 40
<210> 25
   <211> 66
   <212> DNA
   <213> Artificial Sequence
<220>
   <223> Description of Artificial Sequence: Synthetic oligonucleotide
<400> 25
   agatggagac tcgttgtagc tagccccctt gagggcagat tggcgcccga acagggactt 60
   gaagga                                                            66
<210> 26
   <211> 72
   <212> DNA
   <213> Artificial Sequence
<220>
   <223> Description of Artificial Sequence: Synthetic oligonucleotide
<400> 26
   gatctccttc aagtccctgt tcgggcgcca atctgccctc aagggggcta gctacaacga 60
   gtctccatct at                                                      72
<210> 27
   <211> 27
   <212> DNA
   <213> Artificial Sequence
<220>
   <223> Description of Artificial Sequence: Synthetic oligonucleotide
<400> 27
   ccggatctag accgcaagct tcaccgc 27
<210> 28
   <211> 21
   <212> DNA
   <213> Artificial Sequence
<220>
   <223> Description of Artificial Sequence: Synthetic oligonucleotide
<400> 28
   ggtgaagctt gcggtctaga t 21
<210> 29
   <211> 51
   <212> DNA
   <213> Artificial Sequence
<220>
   <223> Description of Artificial Sequence: Synthetic oligonucleotide
<400> 29
   ggccttgaag agcggccgca ctaacaccac cacagtgcgg ccgctcttca a 51
<210> 30
   <211> 51
   <212> DNA
   <213> Artificial Sequence
<220>
   <223> Description of Artificial Sequence: Synthetic oligonucleotide
<400> 30
   ggccttgaag agcggccgca ctgtggtggt gttagtgcgg ccgctcttca a 51
<210> 31
   <211> 121
   <212> DNA
   <213> Artificial Sequence
<220>
   <223> Description of Artificial Sequence: Synthetic oligonucleotide
<400> 31
   atatctatta attttggcaa atcatagcgg ttatgctgac tcaggtgaat gccgcgataa 60
   ttttcagatt gcaatctttc atcaatgaat ttcagtgatg aattgccaag attgatgttg 120
   c                                                                 121
<210> 32
   <211> 111
   <212> DNA
   <213> Artificial Sequence
<220>
   <223> Description of Artificial Sequence: Synthetic oligonucleotide
<400> 32
   gacgagatct cctccaggaa ttctcgagaa ttcggatccc ccgctcccca ccaccaccac 60
   caccaccctg ccccgcggat gaaaaattat gtgagcaaca tcaatcttgg c          111
<210> 33
   <211> 29
   <212> DNA
   <213> Artificial Sequence
<220>
   <223> Description of Artificial Sequence: Synthetic oligonucleotide
<400> 33
   ggtcggcggc cttgaagagc ggccgcact 29
<210> 34
   <211> 42
   <212> DNA
   <213> Artificial Sequence
<220>
   <223> Description of Artificial Sequence: Synthetic oligonucleotide
<220>
   <223> "n" bases represent variable nucleotides
<400> 34
   nnnnnnnnnn nnnrggctag ctacaacgan nnnnnnnnnn nn 42
<210> 35
   <211> 193
   <212> DNA
   <213> Artificial Sequence
<220>
   <223> Description of Artificial Sequence: Synthetic oligonucleotide
<400> 35
   taatacgact cactataggg agacccaagc tggctagcgt ttaaacttaa gcttggtcgg 60
   cggccttgaa gagcggccgc actcacgata gagtgggaga tgggcgcgag aaagtgcggc 120
   cgctcttcaa cctggccgct cgagtctaga gggcccgttt aaacccgctg atcagcctcg 180
   actgtgcctt cta                                                    193
<210> 36
   <211> 129
   <212> DNA
   <213> Artificial Sequence
<220>
   <223> Description of Artificial Sequence: Synthetic oligonucleotide
<400> 36
   ctaggtcggc ggccttgaag agcggccgca ctaacaccac cacagtgcgg ccgctcttca 60
   aggccgccga cccgtcagcg ggggtctttc atttgggggc tcgtccggga tcgggagacc 120
   cctgcccag                                                        129
<210> 37
   <211> 61
   <212> DNA
   <213> Artificial Sequence
<220>
   <223> Description of Artificial Sequence: Synthetic oligonucleotide
<400> 37
   ttagggttag ggttagggtt agggttaggg ttagggttag ggttagggtt agggttaggg 60
   c                                                                 61
<210> 38
   <211> 139
   <212> DNA
   <213> Artificial Sequence
<220>
   <223> Description of Artificial Sequence: Synthetic oligonucleotide
<400> 38
   ctaggtcggc ggccggaaga ttggggcgcc aaagagtaac tctcaaaggc acgcgcccca 60
   atcttccggc cgccgacccg tcagcggggg tctttcattt gggggctcgt ccgggatcgg 120
   gagacccctg cccagggcc                                             139
<210> 39
   <211> 174
   <212> DNA
   <213> Artificial Sequence
<220>
   <223> Description of Artificial Sequence: Synthetic oligonucleotide
<400> 39
   ctaggtcggc ggccggaaga ttggggcgtt agggttaggg ttagggttag ggttagggtt 60
   agggttaggg ttagggttag ggttagggcg ccccaatctt ccggccgccg acccgtcagc 120
   gggggtcttt catttggggg ctcgtccggg atcgggagac ccctgcccag ggcc       174
<210> 40
   <211> 252
   <212> DNA
   <213> Artificial Sequence
<220>
   <223> Description of Artificial Sequence: Synthetic oligonucleotide
<400> 40
   ccccacccgg atctagactc gagcggccag gggtctcccg atcccggacg agcccccaaa 60
   aaggatcccc cgctgactta attaaggtcg gcggccttga agagcggccg cactttctcg 120
   cgcccatctc ccactctatc gtgagtgcgg ccgctcttca aggccgccga ccaagcttca 180
   ccgcggggca gggtggtggt ggtggtggtg gggagcgggg gatccgaatt ctcgagaatt 240
   cctggaggag at                                                     252
<210> 41
   <211> 250
   <212> DNA
   <213> Artificial Sequence
<220>
   <223> Description of Artificial Sequence: Synthetic oligonucleotide
<400> 41
   ggctagcgtt taaacttaag cttggtcggc ggccttgaag agcggccgca ctcacgatag 60
   agtgggagat gggcgcgaga aagtgcggcc gctcttcaag gccgccgacc ttaatggtgg 120
   gcgcctcgtt gtagctagcc tcggtgtggg gatccttttt gggggctcgt ccgggatcgg 180
   gagacccctg gccgctcgag tctagagggc ccgtttaaac ccgctgatca gcctcgactg 240
   tgccttctag                                                        250
<210> 42
   <211> 249
   <212> DNA
   <213> Artificial Sequence
<220>
   <223> Description of Artificial Sequence: Synthetic oligonucleotide
<400> 42
   ggctagcgtt taaacttaag cttggtcggc ggccttgaag agcggccgca ctcacgatag 60
   agtgggagat gggcgcgaga aagtgcggcc gctcttcaag gccgccgacc ttaataatgc 120
   atgtctcgtt gtagctagcc caggcgggag atcctttttg ggggctcgtc cgggatcggg 180
   agacccctgg ccgctcgagt ctagagggcc cgtttaaacc cgctgatcag cctcgactgt 240
   gccttctag                                                         249
<210> 43
   <211> 245
   <212> DNA
   <213> Artificial Sequence
<220>
   <223> Description of Artificial Sequence: Synthetic oligonucleotide
<400> 43
   ggctagcgtt taaacttaag cttggtcggc ggccttgaag agcggccgca ctcacgatag 60
   agtgggagat gggcgcgaga aagtgcggcc gctcttcaag gccgccgacc ttaatgatgt 120
   aagtcgttgt agctagcctc ccctggatcc tttttggggg ctcgtccggg atcgggagac 180
   ccctggccgc tcgagtctag agggcccgtt taaacccgct gatcagcctc gactgtgcct 240
   tctag                                                             245
<210> 44
   <211> 281
   <212> DNA
   <213> Artificial Sequence
<220>
   <223> Description of Artificial Sequence: Synthetic oligonucleotide
<400> 44
   ggctagcgtt taaacttaag cttggtcggc ggccttgaag agcggccgca ctcacgatag 60
   agtgggagat gggcgcgaga aagtgcggcc gctcttcaag gccgccgacc ttaatagatg 120
   gagactcgtt gtagctagcc cccttgaggg cagattggcg cccgaacagg gacttgaagg 180
   agatcctttt tgggggctcg tccgggatcg ggagacccct ggccgctcga gtctagaggg 240
   cccgtttaaa cccgctgatc agcctcgact gtgccttcta g                     281
<210> 45
   <211> 15
   <212> DNA
   <213> Artificial Sequence
<220>
   <223> Description of Artificial Sequence: Synthetic oligonucleotide
<400> 45
   ggctagctac aacga 15

## Description

The present invention relates to a stable DNA construct, conveniently referred to as a cassette, into which a nucleic acid sequence is incorporated for use as a template for subsequent production of that sequence in a prokaryotic or eukaryotic host cell, and a vector systems for expression of that sequence within eukaryotic host cells without (or with minimal) flanking sequences. The cassette includes inverted an tandem repeat which forms the stem of a stem-loop intermediate that functions *in vivo* to cause expression of the sequence as a single stranded DNA (ssDNA) sequence, referred to as the sequence of interest. The vector system of the present invention removes contiguous plasmid (or other vector sequences) from the ssDNA sequence of interest either by stem-loop formation with subsequent termination of a reverse transcription reaction by the stem or by cleavage of the stem-loop intermediate. The ssDNA that is produced by this method can be designed so that it is complimentary to any endogenous nucleic acid sequence target.

So far as is known, no method is available for producing single-stranded deoxyribonucleic acid (ssDNA) species in eukaryotic cells which do not contain intervening and/or flanking vector sequences. The scientific and patent literature does include the disclosure of cDNA-producing vectors (*see* A. Ohshima, *et al.,* 89 Proc. Natl. Acad. Sci. USA 1016-1020 (1992); S. Inouye, *et al.,* 3 Current Opin. Genet. Develop. 713-718 (1993); O. Mirochnitchenko, *et al.,* 269 J. Biol. Chem. 2380-2383 (1994); J.-R. Mao, *et al.,* 270 J. Biol. Chem. 19684-19687 (1995); and U.S. Patent Nos. 5,436,141 and 5,714,323), but the systems disclosed in these references do not appear to have demonstrated the ability to produce ssDNA in eukaryotic cells without intervening vector sequences that can interfere with the intended function of the ssDNA product.

It is therefore an object of the present invention to provide a method for producing single-stranded nucleic acid in yeast, prokaryotic, and/or eukaryotic cells which overcomes this limitation by providing a method, and a DNA construct which directs the synthesis of ssDNA of any desired nucleotide sequence *in vivo* without undesirable intervening or flanking nucleotide bases. The ssDNA can be (but is not limited to) an inhibitory nucleic acid such as an antisense sequence for binding to mRNA in an anti-sense fashion to down regulate a gene product or a viral gene product of interest or for binding to duplex (native DNA) to form triplex structures which may interfere with normal gene transcription and regulation. ssDNA produced in this manner may also function to disrupt one or more of many highly regulated cell functions. For instance, the ssDNA tails of telomeric repeats may be altered by the production of ssDNA which has identical or complimentary nucleotide base composition to the sequence of the native DNA in the telomeric repeats or other regulatory sequence.

This object, and the many others which will be made apparent to those skilled in the art by the following description of several embodiments of the invention, is achieved by providing a cassette, or nucleic acid construct, comprised of a nucleic acid sequence which is comprised of a sequence of interest flanked by inverted tandem repeats, a gene encoding an RNA-dependent DNA polymerase and a gene encoding a restriction endonuclease. The cassette also preferably includes a gene encoding an RNase H and either constitutive or inducible eukaryotic promoter(s)/enhancer(s) for the RNA-dependent DNA polymerase and restriction endonuclease genes. The invention also contemplates that the cassette is incorporated into a plasmid and that the plasmid is incorporated into a suitable host cell.

In another aspect, the present invention comprises a method of producing single-stranded DNA *in vivo* comprising the steps of transcription and translation of a cassette comprising am RNA-dependent DNA polymerase gene and a sequence of interest in a eukaryotic cell and converting the mRNA transcript of the sequence of interest to cDNA with the polymerase produced by the RNA-dependent DNA polymerase gene with simultaneous digestion of the mRNA component template with an RNase H expressed enzyme. The sequence of interest also includes an inverted tandem repeat. The cassette may also include a restriction endonuclease gene which, when transcribed and translated, produces a restriction endonuclease which linearizes the transcript of the sequence of interest by cutting the ss-DNA at a restriction endonuclease site formed when the inverted tandem repeat causes the transcript to form a stem-loop intermediate.

In another aspect, the present invention comprises a method for producing a single-stranded oligonucleotide in a target cell. In one embodiment, this method is intended to deliver an anti-sense sequence. In other embodiments, the method is used to deliver triplex-forming sequences or sequences which are recognized and bound by specific DNA-binding proteins, or other nucleic acids and/or proteins which function in cellular metabolism and/or replication.

The method comprises the encoding of the oligonucleotide into a complementary sequence of interest in a cassette which includes a gene encoding for an RNA-dependent DNA polymerase which preferably includes an RNase H gene and an inducible or constitutive eukaryotic promoter/enhancer appropriate for that polymerase/RNase H gene. The cassette includes a gene encoding a restriction endonuclease (RE) and, in the preferred embodiment, an appropriate promoter/enhancer for that RE gene. The cassette further comprises an inverted tandem repeat and, when assimilated into the target cell, the cassette (including the sequence of interest and the inverted tandem repeats) is transcribed by the cell under the control of the promoter(s)/enhancer(s). The normal function of the target cell causes the resulting mRNA transcript of the polymerase and RE genes to be translated, providing all that is needed for production of ss-DNA from the mRNA transcript of the sequence of interest. Specifically, the RNA-dependent DNA polymerase produced from the cassette converts the mRNA transcript of the sequence of interest and inverted tandem repeats to ss-cDNA, the ss-cDNA forms a stem-loop intermediate as the nucleotide bases comprising the inverted tandem repeats pair up, and the restriction endonuclease produced from the RE gene in the cassette digests the double-stranded portion of the stem-loop intermediate to "free" the single stranded DNA oligonucleotide from the loop portion of the stem-loop intermediate.

Multiple systems can be used to deliver the cassette to the target cell to direct the synthesis of ssDNA within the cell, including plasmid or plasmid-based vector systems or viral based vector systems, and these systems are adapted for that purpose in accordance with standard delivery techniques currently known to the skilled practitioner. These systems include, but are not limited to, viral based systems such as adenovirus, adenoassociated virus, retroviral vectors, and conjugate vectors using double stranded plasma DNA based transfection systems. Once inside the cell, the cassette is transcribed in the normal course of cell metabolism, producing an mRNA transcript of the sequence of interest that is then converted to cDNA by the reverse transcriptase which is likewise produced by the cell from the reverse transcriptase/RNase H gene included in the cassette under the control of the promoter.

According to the present invention, there is provided a nucleic acid construct which comprises an inverted tandem repeat, a primer binding site for a reverse transcriptase located in a 3' position with respect to the inverted tandem repeat, and a sequence of interest located either between the inverted tandem repeat, or between the inverted tandem repeat and the 3' primer binding site.

The nucleic acid construct of the present invention allows a single-stranded nucleic acid sequence to be reliably and stably produced in a target cell. In particular, the inverted tandem repeat may be used to create a nucleic acid stem-loop intermediate to produce and/or control the production of ssDNA *in vivo,* with reduced or eliminated contiguous nucleotide sequences.

Because of the arrangement of the nucleic acid construct, with the primer binding site in a position which is 3' to the sequence of interest, there is no limit to the size or type of sequence of interest that may be produced using the nucleic acid construct of the present invention, and the construct may be easily incorporated into a vector for delivery by any desired route to a target cell.

Where the sequence of interest is located between the inverted tandem repeat, the inverted tandem repeat is, preferably, capable of forming a stem-loop intermediate, with said sequence of interest in the loop, and said inverted tandem repeat forming the stem.

An advantage of this stem-loop structure is that the sequence of interest can be released from the loop as a single-stranded cDNA with reduced or eliminated flanking and/or intervening vector sequences, by cutting the loop where it joins at the stem. The ssDNA, thus, obtained is free or substantially free of any intervening and/or flanking nucleotide sequences, e.g. vector nucleotide sequences, that could interfere with the intended function of the ssDNA product.

Where a sequence of interest is located between the inverted tandem repeat, a second sequence of interest may further be provided between the inverted tandem repeat and the 3' primer binding site.

This embodiment of the invention has the advantage that the inverted tandem repeat can be selected to produce stem-loops of different stabilities, whereby variable amounts of read-through of the mRNA transcript, or early termination of transcription, can be achieved, thereby providing a method of regulating the production of two or more sequences of interest by secondary folding of the intermediate mRNA transcript.

Preferably, the inverted tandem repeat comprises one or more specific enzyme recognition sequence(s).

More preferably, the specific enzyme recognition sequence comprises a restriction endonuclease site. The restriction endonuclease site may either be recognised by an endogenous restriction endonuclease or the nucleic acid construct of the present invention may further comprise a gene encoding a restricting endonuclease for that restriction endonuclease site. In the case that the nucleic acid construct further comprises a restriction endonuclease gene, the restriction endonuclease gene is, preferably, located in a 5' position with respect to the inverted tandem repeat.

The specific enzyme recognition sequence may, for example, include either Hind III and Not I, Hind III, or Not I restriction sites. In fact the specific enzyme recognition sequence may be any one of a restriction endonuclease type I, endonuclease type II, endonuclease type III, eukaryotic receptor recognition, prokaryotic receptor recognition, promoter, promoter/enhancer and T-overhang PCR site, or combinations thereof.

Where the sequence of interest is located between the inverted tandem repeat and the 3' primer binding site, or where a first sequence of interest is located between the inverted tandem repeat and a second sequence of interest is located between the inverted tandem repeat and the 3' primer binding site, the inverted tandem repeat is, preferably, capable of forming a stable stem-loop, an unstable stem-loop, or a stem-loop of intermediate stability.

The inverted tandem repeats are selected to form a stable stem-loop in the mRNA transcript as a means of causing premature termination of reverse transcription, so that if a sequence of interest is located in the mRNA transcript between the start site for reverse transcription and the stem-loop structure, ssDNA substantially free of contiguous vector sequences can again be produced.

The inverted tandem repeat(s) of the present invention allow the use of a stem-loop structure as a vehicle for removing unwanted contiguous nucleotide sequences, for example, either by causing premature termination of reverse transcription from the mRNA transcripts or by cutting away unwanted sequences from the cDNA via restriction endonuclease sites in the inverted tandem repeats.

According to another preferred embodiment of the present invention, the inverted tandem repeat may comprise one or more eukaryotic, prokaryotic, and or/viral protein DNA binding sites. The stem-loop structure of the present invention, thus, has the further advantage that it can also serve as a functional entity in itself, e.g. to protect the ssDNA in the loop from degradation by intracellular nucleases, or to carry out certain applications via functional elements (e.g. protein binding sites) in the inverted tandem repeats.

Preferably, the inverted tandem repeat acts in *cis*-orientated fashion.

The primer binding site may be specific for an endogenous reverse transcriptase (e.g. in the case of a cell infected by human immunodeficiency virus or simian immunodeficiency virus).

Alternatively, the nucleic acid construct may further comprise a gene encoding a reverse transcriptase. The reverse transcriptase gene is, preferably, located in a 5' position, with respect to the inverted tandem repeat.

Alternatively, the nucleic acid construct may further comprise a gene encoding a reverse transcriptase/RNAse H polyprotein. The reverse transcriptase/RNAse H polyprotein gene is, preferably, located in a 5' position with respect to the inverted tandem repeat. The reverse transcriptase/RNAse H polyprotein gene may be derived from Moloney murine leukaemia virus, human immunodeficiency virus, or simian immunodeficiency virus.

In the case that the nucleic acid construct of the present invention is provided with a reverse transcriptase or reverse transcriptase/RNAse H polyprotein gene, the primer binding site is, preferably, specific for the reverse transcriptase or reverse transcriptase/RNAse H polyprotein encoded by their respective genes.

Preferably, the nucleic acid of the present invention further comprises a promoter and, optionally, an enhancer for each of said first of second sequence of interest, said restriction endonuclease, said reverse transcriptase and/or said reverse transcriptase/RNAse H gene.

More preferably, the promoter and/or enhancer is a eukaryotic promoter/enhancer.

The promoter may be a constitutive, inducible wide-spectrum or tissue-specific promoter.

Preferably, the nucleic acid construct of the present invention further comprises a polyadenylation tail sequence located in a 3' position with respect to the 3' primer binding site. The polyA tail provides for stabilisation of the mRNA transcript.

Preferably, the first or second sequence of interest includes a sequence which encodes a single-stranded DNA having enzymatic activity. One example of such a single stranded DNA having enzymatic activity comprises the sequence 5' - GGCTAGCTACAACGA-3', which has RNAse activity. This sequence is flanked in both the 5' and 3' directions by one or more sequence(s) which are complementary to a target mRNA species, for example, h-ras, c-raf kinase, antisense sequence to pleiotrophin angiogenic growth factor, or the tat region of simian immunodeficiency virus.

Preferably, the primer binding site is complementary to a transfer RNA (tRNA).

Preferably, the nucleic acid construct is DNA.

The nucleic acid construct of the present invention, thus, provides an efficient system for directing the synthesis of a stable, single-stranded nucleic acid sequence, both *in vivo* and *in vitro.* The single-stranded nucleic acid sequence may be used to provide a desired effect in a cell, tissue or organism. Because production of the single-stranded nucleic acid sequence of interest takes place within the cell, prior art problems arising from delivery of the single-stranded nucleic acid sequence to the cell are overcome, or at least alleviated.

Also provided is an mRNA transcript of the nucleic acid construct of the present invention.

According to another aspect of the present invention, there is provided an mRNA transcript comprising a sequence of interest flanked by an inverted tandem repeat, and further comprising a primer binding site located 3' to the inverted tandem repeat.

According to this aspect of the present invention, there is also provided an mRNA transcript comprising an inverted tandem repeat and a primer binding site located 3' to the inverted tandem repeat, and a sequence of interest located between the inverted tandem repeat and the 3' primer binding site.

According to this aspect of the present invention, there is further provided an mRNA transcript comprising a first sequence of interest flanked by an inverted tandem repeat, a primer binding site located 3' with respect to the inverted tandem repeat, and a second sequence of interest between the inverted tandem repeat and 3' primer binding site.

Further provided is a single stranded DNA transcript of the mRNA of the present invention.

Also provided is a vector comprising a nucleic acid construct according to the present invention.

The nucleic acid constructs of the present invention are such that they may be incorporated into commercially available delivery vectors for mammalian and human therapeutic purposes, and may be administered by any feasible route, depending on the target cell.

According to the present invention there is also provided a vector which comprises an inverted tandem repeat, a primer binding site for a reverse transcriptase located in a 3' position with respect to the inverted tandem repeat, and an insertion site for a sequence of interest between the inverted tandem repeat, or between the inverted tandem repeat and the primer binding site.

Preferably, the vector comprises a first insertion site between the inverted tandem repeat and a second insertion site between the inverted tandem repeat and the 3' primer binding site. This allows a user to insert any desired sequence of interest into either, or both, the insertion sites. Because of the arrangement of the nucleic acid construct, with the primer binding site in a position which is 3' to the inverted tandem repeat, there is no limit on the size or type of sequence of interest that may be inserted into the insertion site.

Preferably, the vector further comprises a gene encoding a reverse transcriptase, or a gene encoding a reverse transcriptase/RNAse H polyprotein. Preferably, the reverse transcriptase or reverse transcriptase/RNAse H polyprotein gene is located in a 5' position with respect to the inverted tandem repeat.

According to the present invention, there is further provided a vector system, which comprises a first vector according to the present invention, (i.e. a vector which comprises an inverted tandem repeat, a primer binding site for a reverse transcriptase located in a 3' position with respect to the inverted tandem repeat, and an insertion site for a sequence of interest between the inverted tandem repeat, or between the inverted tandem repeat and the primer binding site) and a second vector, which comprises a gene encoding a reverse transcriptase, a reverse transcriptase/RNAse H polyprotein, or a reverse transcriptase/RNAse H polyprotein linked via a proline rich linker to a restriction endonuclease.

The vector or vectors systems of the present invention may also be designed to allow the primer binding site to be removed and exchanged, so that different primer binding sites can be used, depending upon the requirements of the user and the specificity of the reverse transcriptase being used.

Preferably, the vector or vector system of the present invention is a plasmid or modified viral construct.

Preferably, the reverse transcriptase, reverse transcriptase/RNAse H polyprotein, or reverse transcriptase/RNAse H/restriction endonuclease gene is operably linked to an expression control sequence.

The vector or vector systems of the present invention may be advantageously employed to deliver antisense, sense, triplex, or any other single-stranded nucleotide sequence of interest into a cell, using known digestion and ligation techniques to splice the sequence of interest into the vector. The vector or vector system described herein provides all the necessary signalling instructions and enzymatic functions to allow a host cell to produce a single-stranded nucleic acid molecule having a desired sequence.

Also provided is a host cell stably transformed or transfected with a vector or vector system according to the present invention. In particular, the host cell may be a eukaryotic or prokaryotic cell. Eukaryotic cells include yeast, plant and mammalian cells. Prokaryotic cells include bacterial cells.

According to the present invention there is further provided a kit for producing a single-stranded nucleic acid sequence, which kit comprises a vector or vector system according to the present invention, and a restriction endonuclease for each insertion site.

According to another aspect of the present invention, there is provided a kit for producing a single-stranded nucleic acid sequence, which kit comprises a vector or vector system according to the present invention, a container for the vector/vector system, and instructions for use of the vector/vector system.

According to the present invention there is further provided an *in vivo* or *in vitro* method of producing a single-stranded nucleic acid sequence of interest, which method comprises the steps of introducing a nucleic acid construct according to the present invention into a target cell, transcribing the nucleic acid construct into mRNA and reverse transcribing the mRNA transcript into cDNA.

Preferably, the method further comprises the step of removing the mRNA transcript from an mRNA/cDNA heteroduplex formed by reverse transcription of the mRNA.

Reverse transcription may be carried out either by a reverse transcriptase which is endogenous to the target cell, or the method of the present invention may further comprise the step of introducing a gene encoding a reverse transcriptase, or a gene encoding a reverse transcriptase/RNAse H polyprotein, into the target cell.

Preferably, the method of the present invention further comprises the step of linearising the cDNA transcript of the sequence of interest by cutting the cDNA stem loop structure formed by the inverted tandem repeat where the loop structure joins the stem.

According to this latter preferred embodiment of the present invention, the method further comprises the step of introducing a gene encoding a restriction endonuclease into the target cell. By providing one or more restriction endonuclease sites in the inverted tandem repeat, the restriction endonuclease expressed by the restriction endonuclease gene can then be used to cut the cDNA stem loop structure. Alternatively, cutting of the cDNA stem loop structure may rely on restriction endonucleases which are endogenous to the target cell.

The restriction endonuclease may also be provided by the step of introducing a gene encoding a reverse transcriptase/RNAse H polyprotein linked via a proline rich linker to a restriction endonuclease into the target cell.

Where the single-stranded nucleic acid sequence is prepared by an *in vitro* method of the present invention, the method may further comprise the step of isolating the mRNA transcript, mRNA/cDNA heteroduplex and/or single-stranded cDNA from the target cell.

Also provided is a single-stranded cDNA transcript, an inhibitory nucleic acid molecule (e.g. an antisense sequence or aptamer), a single-stranded DNA having enzymatic activity, (e.g. RNAse activity), an mRNA transcript and/or a heteroduplex molecule produced by the methods of the present invention.

An inhibitory nucleic acid molecule may be single-stranded DNA synthesized from the mRNA transcript, or the mRNA transcript itself, which can specifically bind to a complementary nucleic acid sequence. Such inhibitory nucleic acid molecules may be "sense" or "antisense" sequences, and are particularly useful for regulating gene function. An inhibitory nucleic acid molecule may also be an oligo-nucleotide that specifically binds to an RNA or DNA-binding protein, or an oligo-nucleotide that binds to a biomolecule, e.g. thrombin, bradykinin or PGF2α, which does not normally bind to RNA or DNA.

According to another aspect of the present invention, there is provided a pharmaceutical composition which comprises a nucleic acid construct, vector or vector system, or host cell according to the present invention, together with a pharmacologically acceptable adjuvant, diluent or carrier.

According to the present invention, there is provided a nucleic acid construct, vector or vector system, or host cell according to the present invention for use in therapy, especially for use in delivering an inhibitory nucleic acid molecule to a target cell. The nucleic acid construct, vector or vector system and host cell of the present invention are particularly useful for alleviating a pathological condition by regulating gene expression.

According to the another aspect of the present invention, there is further provided the use of a nucleic acid construct, vector or vector system, or host cell according to the present invention for the manufacture of a medicament for alleviating a pathological condition by regulating gene expression, especially for alleviating a pathological condition by delivery of an inhibitory nucleic acid molecule to a target cell. Other uses are also disclosed.

The sets of genetic elements, vectors, vector systems and host cells of the present invention may be used for the prophylactic or therapeutic treatment of a wide range of conditions or diseases, particularly conditions or diseases which are caused by abnormal or altered gene expression, or conditions or diseases which may be alleviated by regulating gene expression.

The sets of genetic elements, vectors, host cells, kits and methods of the present invention may be used to produce single-stranded nucleic acid molecules or virtually any predefined or desired nucleotide base composition in a host cell, and are adaptable and applicable to any *in vivo* or *in vitro* system.

According to a preferred embodiment, the nucleic acid construct of the present invention is an artificially synthesised, recombinant, chimeric, and/or heterologous product, and the sequence of interest may be foreign to the cell in which it is produced.

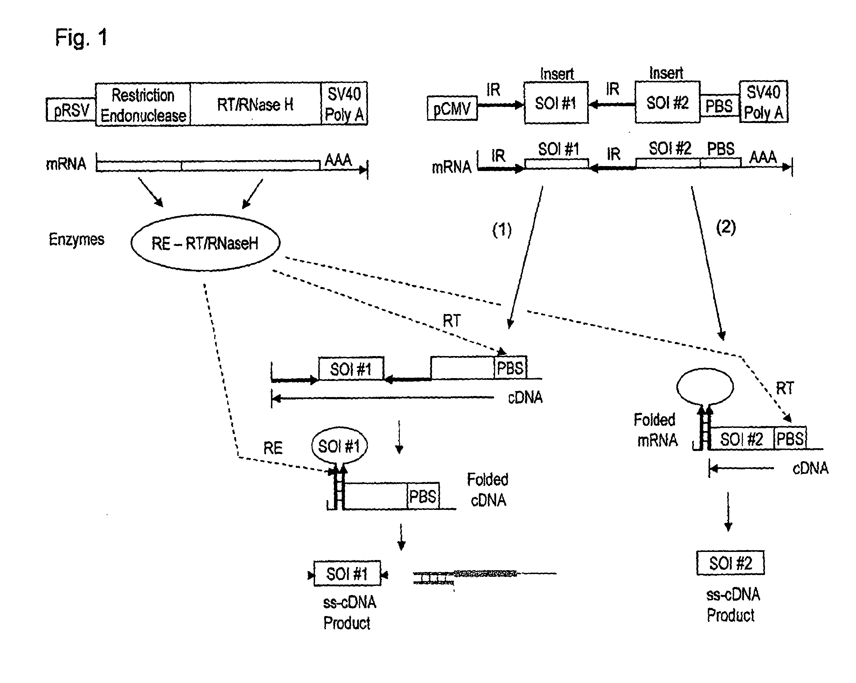

These several embodiments of the invention are illustrated in the figures, in which Figure 1 is a schematic illustration of the production of ss-cDNA in a host cell in accordance with the present invention. The 'loop' embodiment (1) produces a small ss-cDNA complementary to the SOI cloned into the loop position using a restriction-like enzyme to cleave the folded cDNA intermediate. The '3' stem' embodiment (2) produces a similar product when the SOI is cloned 3' of the stem-loop region wherein the folded mRNA stem causes premature truncation of the cDNA during transcription.

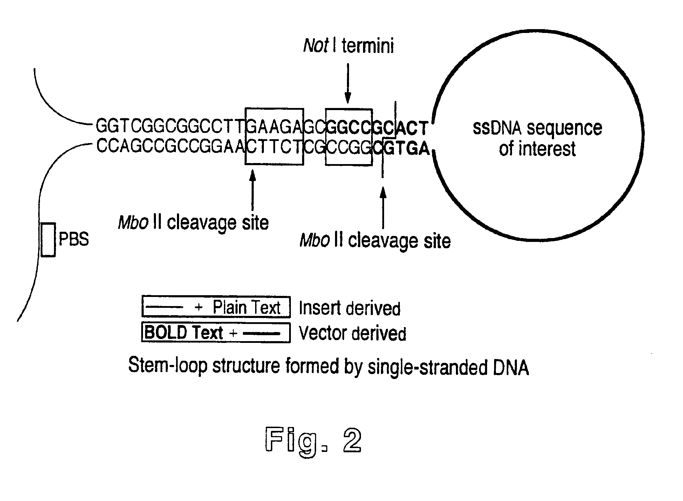

Figure 2 is a schematic illustration of the stem-loop intermediate formed by the method illustrated in Fig. 1.

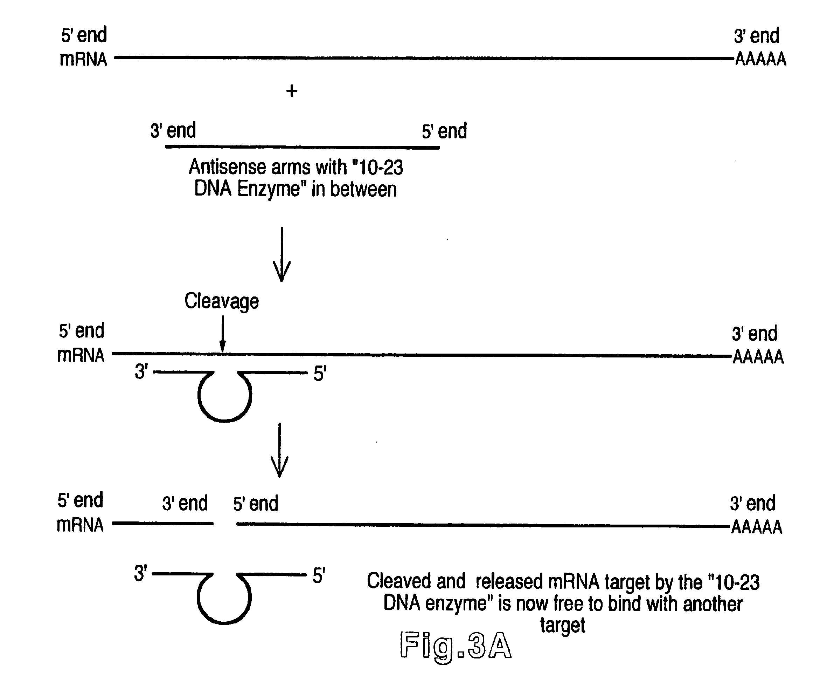

Figure 3A is a schematic representation of the binding of an antisense sequence produced in accordance with the present invention which incorporates the "10-23 DNA enzyme" binding to a target mRNA and the subsequent cleavage of the target mRNA. Figure 3B is an enlarged representation of the binding of the antisense sequence of Fig. 3A to the target mRNA showing the interaction of the 10-23 DNA enzyme (included in a generalized inhibitory nucleic acid sequence) with the cleavage site of the target mRNA.

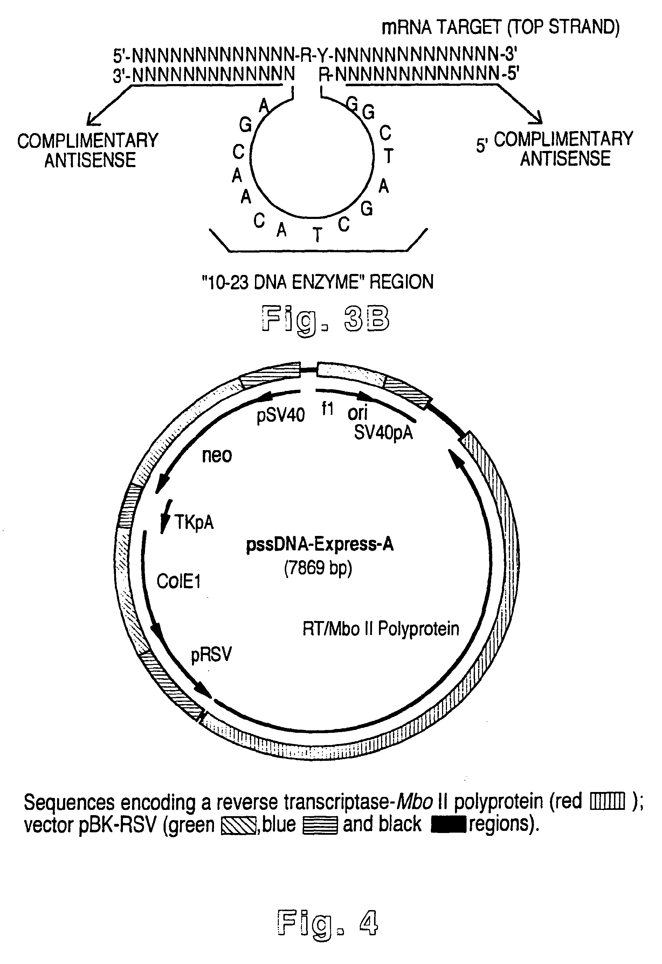

Figure 4 is a schematic map of the plasmid pssDNA-Express A constructed in accordance with the present invention.

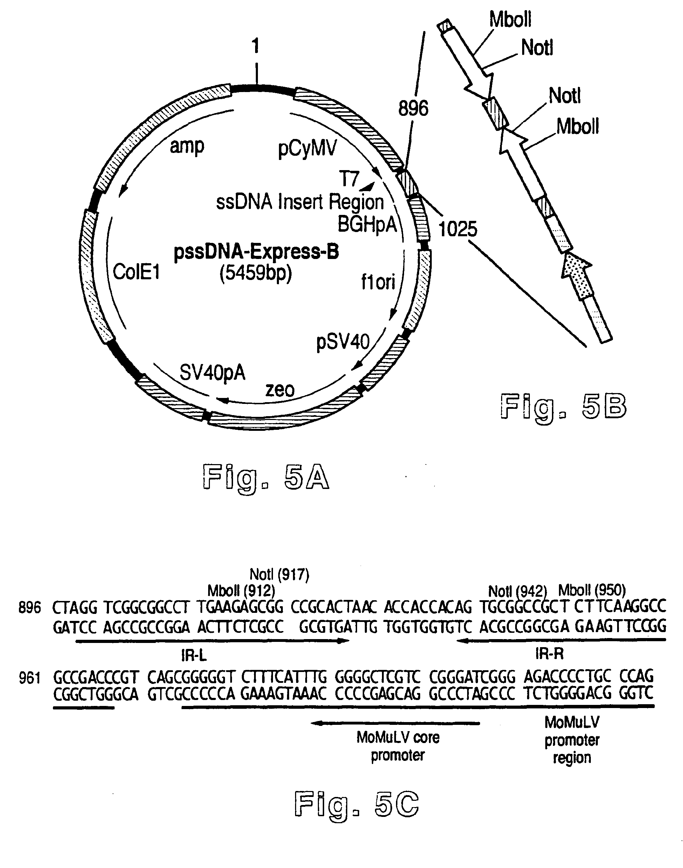

Figures 5A, 5B, and 5C represent a schematic map of the plasmid pssDNA-Express-B constructed in accordance with the present invention, an enlarged portion of the plasmid pssDNA-Express B, and the sequence of the insert region of plasmid pss-DNA-Express B, respectively.

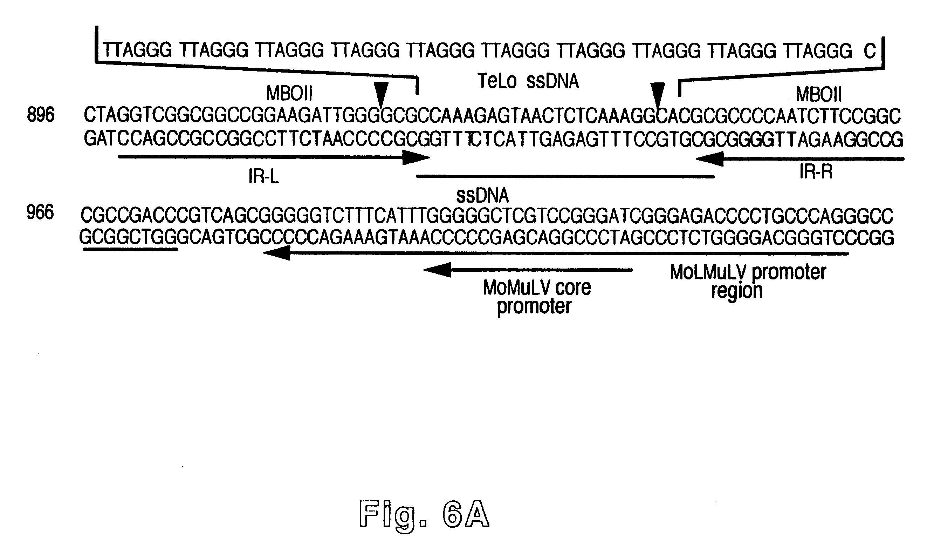

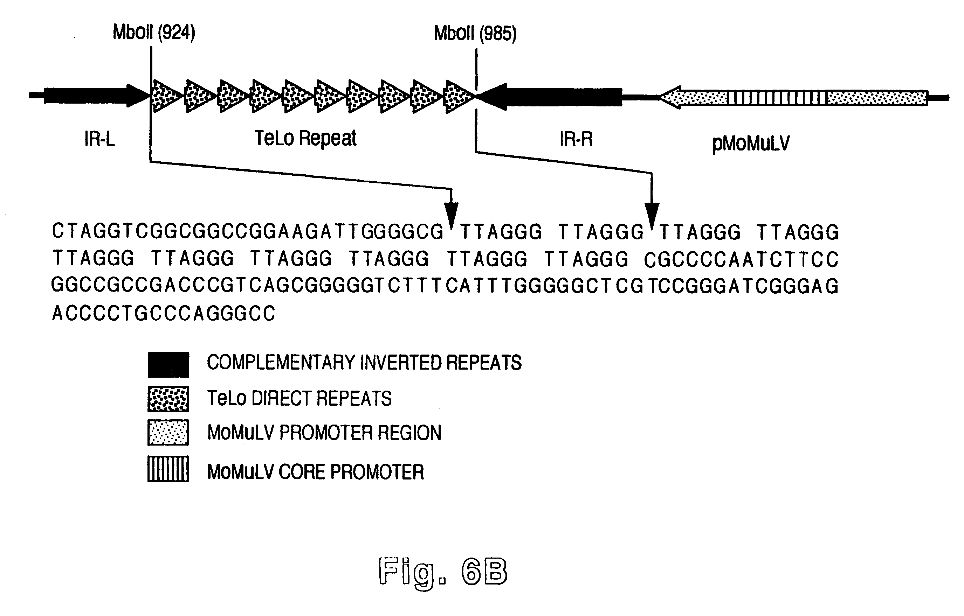

Figures 6A and 6B represent the sequence of the insert regions between the *Apa* I and *Nhe* I sites of the plasmids pTest and pTelo constructed from the plasmid pssDNA-Express-B in accordance with the present invention.

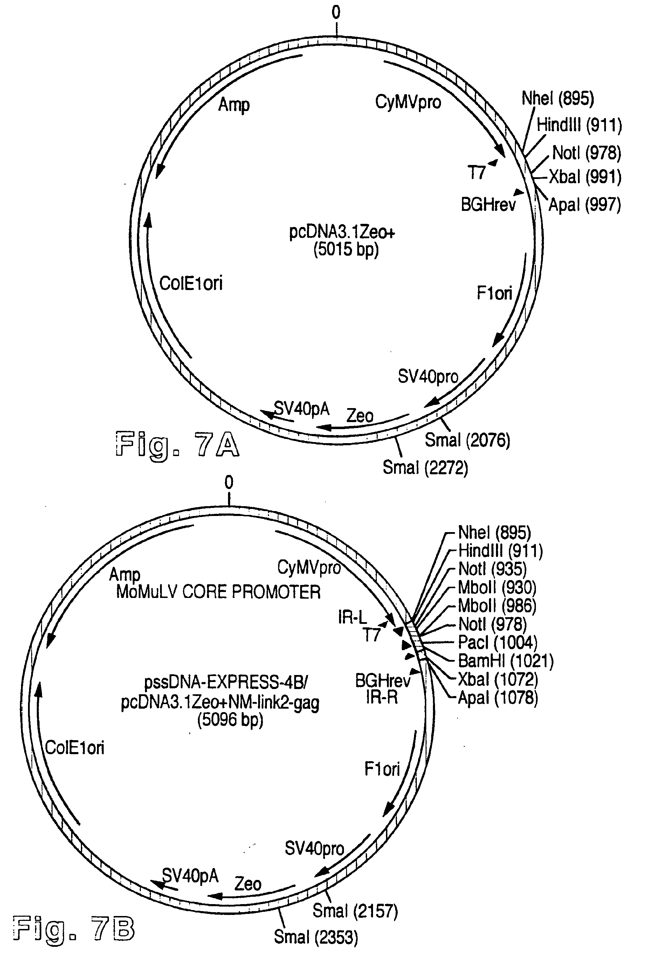

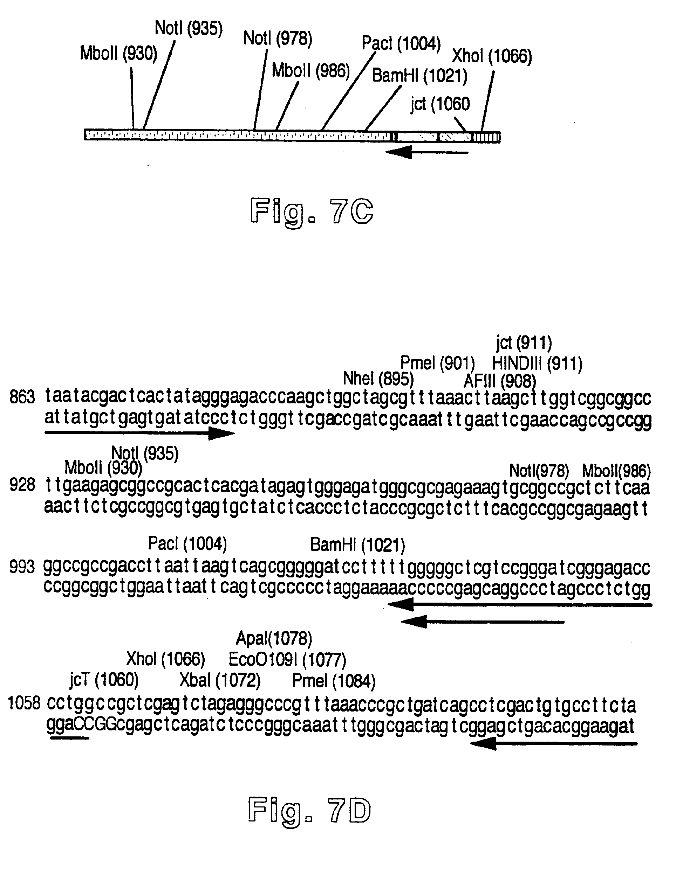

Figure 7A is a schematic map of the starting plasmid pcDNA3.1Zeo⁺ (Invitrogen, Inc.) for plasmid pcDNA3.1Zeo⁺/NM-link2-gag/pssDNA-Express-4B constructed in accordance with the present invention. Figure 7B is a schematic map of pcDNA3.1Zeo⁺/NM-link2-gag (also designated pssDNA-Express-4B). Figure 7C is a schematic representation of the cloning sites, inverted repeats, and PBS region (arrow) for pcDNA3.1Zeo⁺/NM-link2-gag/pssDNA-Express-4B. Figure 7D is a sequence map of the cloning sites, inverted repeats, and PBS region (arrow) for pcDNA3. IZeo⁺/NM-link2-gag/pssDNA-Express-4B.

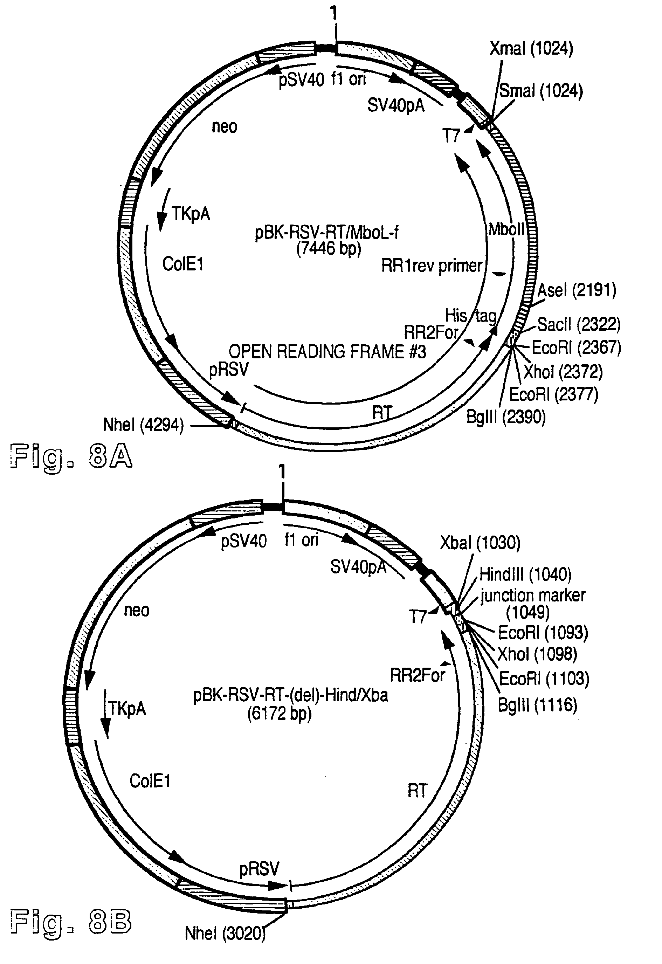

Figure 8A is a schematic map of the plasmid pssDNA-Express A constructed in accordance with the present invention. Figure 8B is a schematic map of the plasmid pssDNA-Express-A after deletion of the Mbo II gene by digesting with Xma I and Sac II.

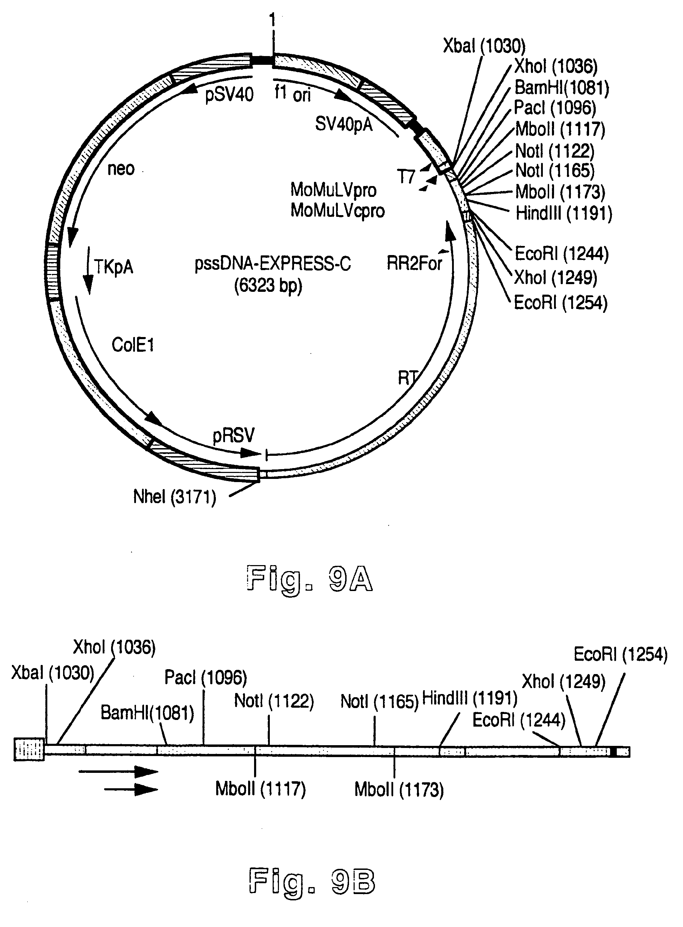

Figure 9A is a schematic map of the plasmid pssDNA-Express-C constructed in accordance with the present invention. Figure 9B is a schematic representation of the cloning sites, inverted repeats, and PBS region (arrow) for plasmid pssDNA-Express-C. Figure 9C is a sequence map of the cloning sites, inverted repeats, and PBS region (arrow) for plasmid pssDNA-Express-C.

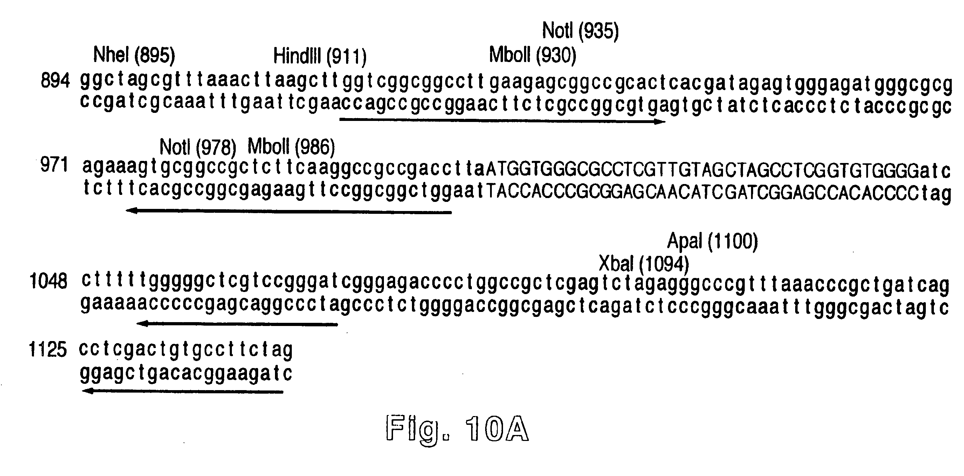

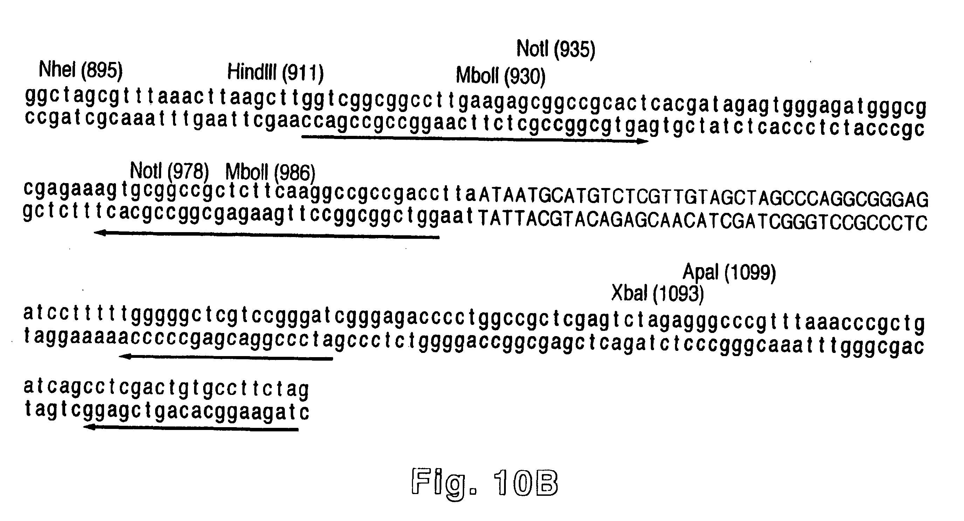

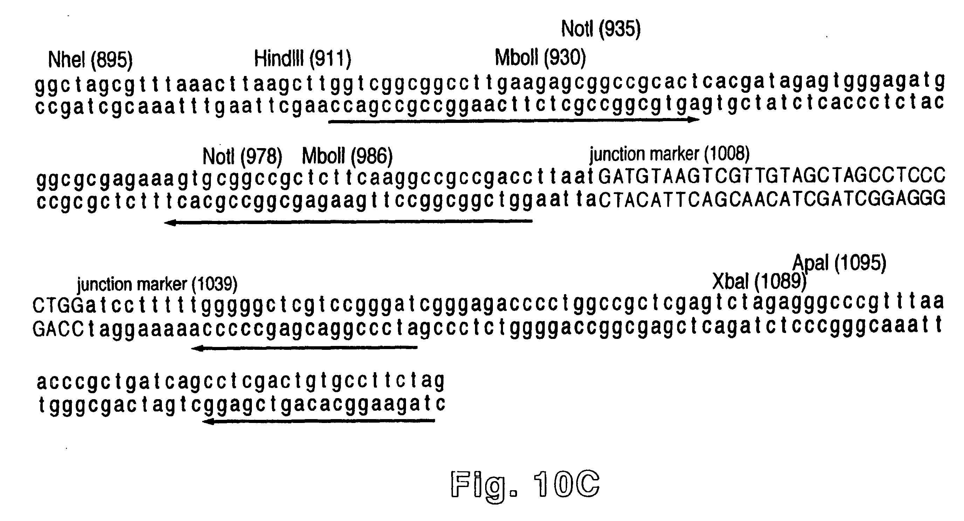

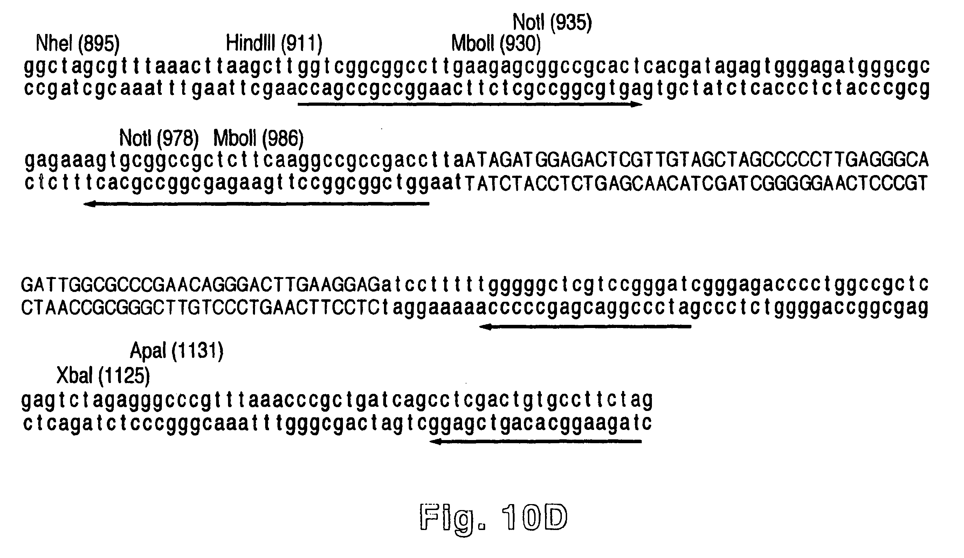

Figure 10A shows the partial sequence of the 23rd codon of h-ras antisense binding sequence with the 10-23 DNA enzyme sequence inserted between the 5' and 3' complimentary sequences. Figure 10B shows the partial sequence of c-raf kinase antisense binding sequence with the 10-23 DNA enzyme sequence inserted between the 5' and 3' complimentary sequences. Figure 10C shows the partial sequence of pleiotrophin antisense binding sequence with the 10-23 DNA enzyme sequence inserted between the 5' and 3' complimentary sequences. Figure 10D shows the partial sequence of tat antisense binding region of the SIV sequence with the 10-23 DNA enzyme sequence inserted between the 5' and 3' complimentary sequences.

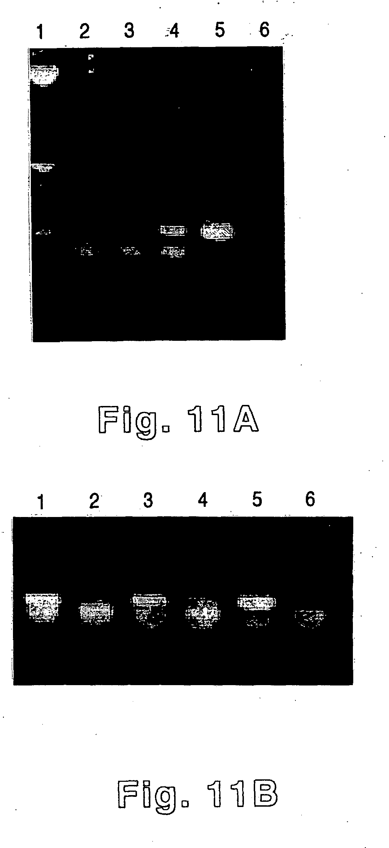

Figures 11A and 11B are gels showing the results of PCR reverse transcriptase assays (J. Silver, *et al.,* 21 Nucleic Acids Res. 3593-3594 (1993)) on RNA/ssDNA extracts from HeLa cell lines transformed with the plasmid pTest constructed in accordance with the present invention. Lanes, Fig. 11A (1) size markers, (2) untransformed HeLa cells, (3) HeLa cells transformed with the vector pcDNA3.1Zeo⁺, (4) HeLa cells transformed with pssXA, (5) positive control containing 2 milliunits of MoMuLV reverse transcriptase, and (6) negative control containing no protein extract. The arrow indicates the 150 bp amplification product. Lanes, Fig. 11B: (1) clone A12, (2) B8, (3) B12, (4) D7, (5) positive control containing 2 milliunits of MoMuLV reverse transcriptase, and (6) negative control containing no protein extract.

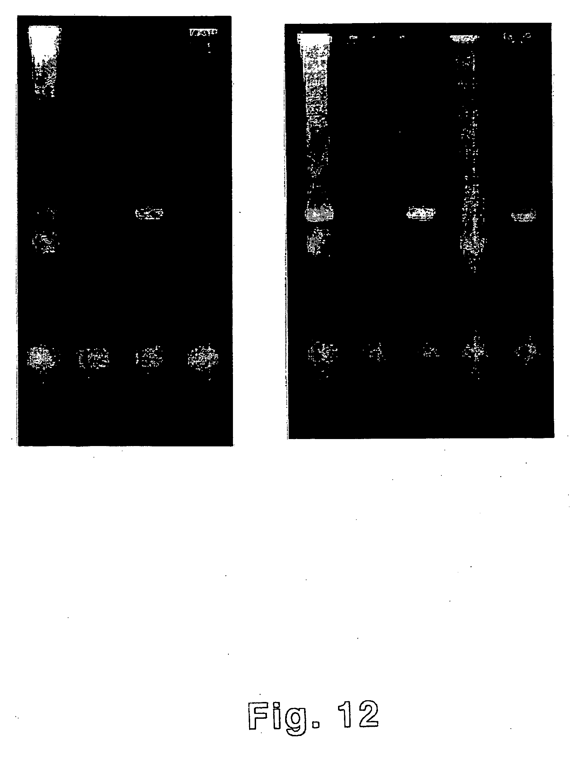

Figure 12 are gels showing PCR amplification of single-stranded DNA from extracts of HeLa cell lines transformed with the plasmid pTest constructed in accordance with the present invention. RNA/ssDNA preparations were used as templates in PCR reactions using primers specific to the expected ssDNA product. Left panel, lanes 1-3: total RNA/ssDNA PCR template isolated from stably transformed colony A12 (HeLa cell line stable transfected with plasmid pssDNA-Express-A) (1) used with no prior treatment, (2) treated with S1 nuclease, (3) treated with RNAse A. Lane (4) total RNA/ssDNA from a colony stably transformed with the pcDNA3.1Zeo⁺ vector alone. Right panel, lanes 1-3: total RNA/ssDNA PCR template isolated from stably transformed colony B12 (HeLa cell line stably transfected with plasmid pssDNA-Express-A) (1) used with no prior treatment, (2) treated with S1 nuclease, (3) treated with RNAse A. Lane (4) total RNA/ssDNA from a colony stably transformed with the pcDNA3.1Zeo⁺ vector alone. Lane (5), positive control template, plasmid pTest.

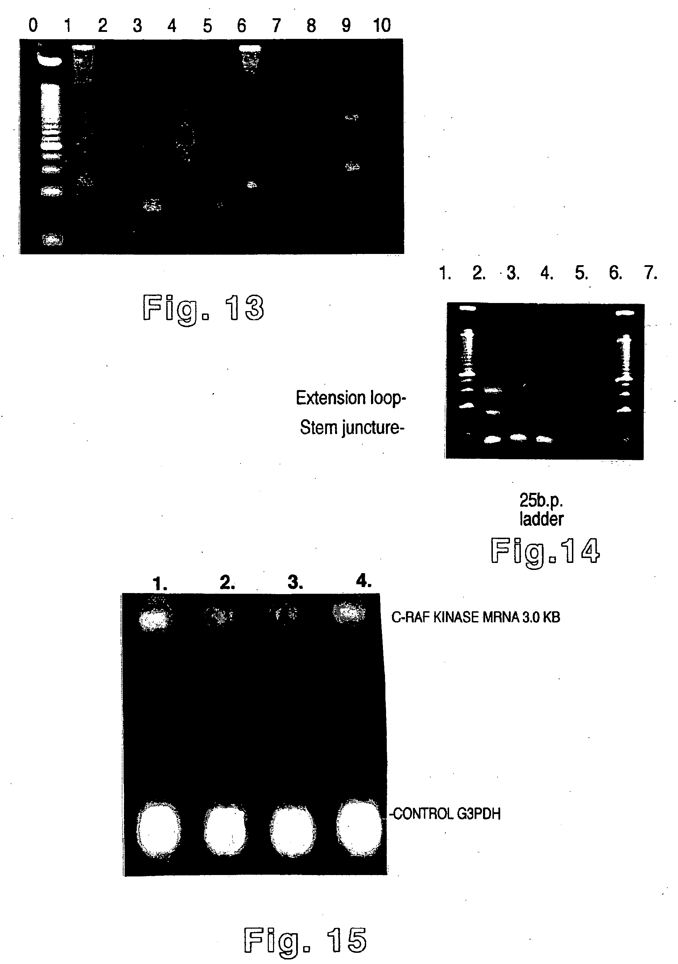

Figure 13 is a gel showing PCR amplification of single-stranded DNA from extracts of cells transformed with the plasmid pTelo constructed in accordance with the present invention. RNA/ssDNA was harvested from transfected cell cultures at 36 hrs post transfection and used as templates for PCR reactions using primers specific to the expected ssDNA product. 25 bp lane marker used. Lanes 1-5 with isolation from HeLa cell line A12, (1) total RNA/ssDNA fraction, (2) total RNA treated with S1 nuclease, (3) total RNA treated with RNAse A, (4) negative control, (5) positive control telomere DNA plasmid. Lanes 6-9 RNA isolated from B12 cell line, (6) total RNA fraction, (7) total RNA treated with S 1 nuclease, (8) total RNA treated with RNAse A, (9) negative control, (10) positive control telomere DNA plasmid. Lanes 11-12 repeat negative controls. 8% acrylamide gel at 45 V for 20 mins.

Figure 14 is a gel showing premature truncation of ss-cDNA transcription and read through products by *in vitro* reverse transcriptase reaction. Lanes 1-5 represent plasmid constructs with stem-loop structures constructed in accordance with the present invention and various sequences of interest cloned within the loop region. RNA template was produced by *in vitro* transcription with T7 RNA polymerase under standard conditions, then treated with DNAse to remove plasmid template. Phenol/chloroform extracted RNA pools were then reverse transcribed with mouse Moloney reverse transcriptase, treated with RNAse A for 15 mins. and resolved on a 6% acrylamide gel at 45 V for 30 mins. 25 bp marker at borders. (1) 10 µl RT reaction from original plasmid pssDNA-Express-B constructed in accordance with the present invention, (2) 10 µl RT reaction from original plasmid pTest constructed in accordance with the present invention, (3) 10 µl RT reaction from original plasmid pTelo constructed in accordance with the present invention, (4) negative control plasmid pcDNA-Zeo (Invitrogen), (5) 3 µl repeat pssDNA-Express-B.

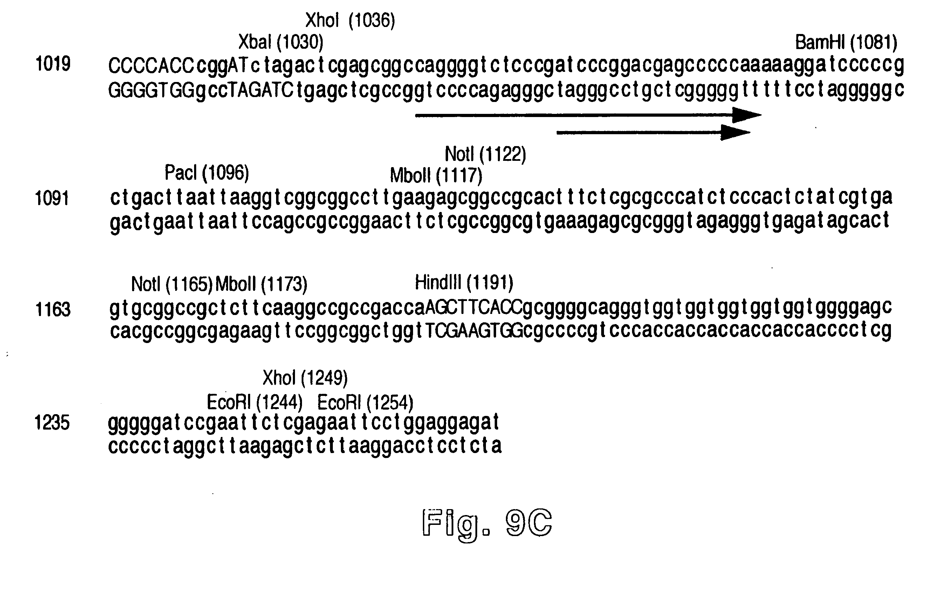

Figure 15 shows a Northern blot of an antisense producing vector constructed in accordance with the present invention producing an antisense sequence against c-raf kinase (Fig. 10B) and including the "10-23 DNA enzyme" after transfection of plasmid pssDNA-Express-C (Fig. 9) including that sequence into a lung cancer cell line *in vitro.*

In this description of the preferred embodiments of the present invention, methods and nucleic acid constructs are described for use in producing single-stranded deoxyribonucleic acid (ss-cDNA) oligonucleotides of virtually any predefined or desired nucleotide base composition *in vivo* in yeast, prokaryotic cells, and/or eukaryotic cells with or without flanking nucleotide sequences. Methods and constructs are described which use biological rather than the *in vitro,* or artificial, synthesis of ss-cDNA of desired nucleotide base composition. Because biological, i.e., enzymatic reactions, are used in these methods they are applicable to any *in vivo* system.

A vector (as used herein, the term "vector" refers to a plasmid or modified viral construct used to deliver and manipulate DNA segments of interest) system was designed to produce any DNA sequence as a ss-cDNA molecule, free of most contiguous vector sequences, within yeast, prokaryotic, and/or eukaryotic cells. The vector system contains all the necessary enzymatic functions and signaling instructions to enable the host cell to which the vector is delivered to produce ss-cDNA. The cell to which the vector of the present invention is delivered produces an RNA transcript (Fig. 1), driven by an eukaryotic promoter just as the above-described enzymes are driven by eukaryotic promoters, which is used as a template to direct the synthesis of any desired single-stranded DNA sequence (a "sequence of interest"). In more detail, a first system is described herein in which the vector comprises two plasmids that are co-transfected into a suitable host cell, which can be yeast or any prokaryotic or eukaryotic cell, to produce a ssDNA including the sequence of interest in the cell. A second system is described in which the vector system comprises a single plasmid containing the sequence of interest that is transfected into a suitable host cell for production of an inhibitory nucleic acid, such as the antisense sequence, from the sequence of interest.

Inhibitory nucleic acids may be ssDNA synthesized from the mRNA template or the mRNA template itself, which can specifically bind to a complementary nucleic acid sequence. By binding to the appropriate target sequence, an RNA--RNA, a DNA--DNA, or RNA-DNA duplex or triplex is formed. These nucleic acids are often termed "antisense" because they are usually complementary to the sense or coding strand of the gene, but the "sense" sequence is also utilized in the cell for therapeutic purposes. For example, the identification of oligonucleotides that specifically bind to biomolecules that do not normally bind to RNA or DNA has now been demonstrated for a number of biomolecules that vary widely in size, structure and composition. Examples of such molecules include (but are not limited to): (1) thrombin, a multifunctional regulatory protein that converts fibrinogen to fibrin in the process of clot formation; (2) bradykinin, a nonapeptide kinin involved in blood pressure regulation and implicated in hypotension; (3) PGF2 alpha, a prostaglandin or fatty acid derivative that exhibits hormonal activity. Additionally, the interaction of oligonucleotides with biomolecules whose natural biological function is primarily extracellular has now been demonstrated (*see,* for instance, U.S. Patent No. 5,840,867). The term "inhibitory nucleic acids" as used herein, therefore, refers to both "sense" and "antisense" nucleic acids.

By binding to the target nucleic acid, an inhibitory nucleic acid inhibits the function of the target nucleic acid. This inhibitory effect results from, for example, blocking DNA transcription, processing or poly(A) addition to mRNA, DNA replication, translation, or promoting inhibitory mechanisms of the cells (such as promoting RNA degradation). Inhibitory nucleic acid methods therefore encompass a number of different approaches to altering expression of genes. These different types of inhibitory nucleic acids are described in Helene, C. and J. Toulme, 1049 Biochim. Biophys. Acta. 99-125 (1990).

In brief, inhibitory nucleic acid therapy approaches can be classified into (1) those that target DNA sequences, (2) those that target RNA sequences (including pre-mRNA and mRNA), (3) those that target proteins (sense strand approaches), and (4) those that cause cleavage or chemical modification of the target nucleic acids such as the ssDNA enzymes, including the so-called "10-23 enzyme" as described herein. The first approach contemplates several categories. Nucleic acids are designed to bind to the major groove of the duplex DNA to form a triple helical or "triplex" structure. Alternatively, inhibitory nucleic acids are designed to bind to regions of single stranded DNA resulting from the opening of the duplex DNA during replication or transcription. More commonly, inhibitory nucleic acids are designed to bind to mRNA or mRNA precursors. Inhibitory nucleic acids are used to prevent maturation of pre-mRNA. Inhibitory nucleic acids may be designed to interfere with RNA processing, splicing or translation. In the second approach, the inhibitory nucleic acids are targeted to mRNA. In this approach, the inhibitory nucleic acids are designed to specifically block translation of the encoded protein. Using this second approach, the inhibitory nucleic acid is used to selectively suppress certain cellular functions by inhibition of translation of mRNA encoding critical proteins. Inhibitory nucleic acids targeting mRNA have been shown to work by several different mechanisms to inhibit translation of the encoded protein(s). An example of such an inhibitory nucleic acid is the sequence that is complementary to regions of c-myc mRNA, which inhibits c-myc protein expression in a human promyelocytic leukemia cell line, HL60, which overexpresses the c-myc proto-oncogene. Wickstrom E. L., *et al.,* 85 Proc. Natl. Acad. Sci. USA 1028-1032 (1988); Harel-Bellan, A., *et al.,* 168 Exp. Med. 2309-2318 (1988).

The inhibitory nucleic acids produced in the cell can also utilize the third approach of designing the "sense" strand of the gene or mRNA to trap or compete for enzymes or binding proteins involved in mRNA translation. Lastly, inhibitory nucleic acids are used to induce chemical inactivation or cleavage of the target genes or mRNA. Chemical inactivation occurs, for instance, by induction of crosslinks between the inhibitory nucleic acid and the target nucleic acid within the cell and by the method contemplated herein, namely, the cleavage of the target mRNA by the sequence having enzymatic activity against mRNA that is incorporated into the cassette of the present invention.

In brief, in a first aspect, the present invention comprises a set of genetic elements adapted for delivery into a cell to produce ss-DNA *in vitro* or *in vivo.* The set of genetic elements is incorporated into at least one vector for delivery into the cell and includes
(a) a RNA dependent DNA polymerase (reverse transcriptase) gene, and
(b) a cassette including (1) an inverted tandem repeat (IR), (2) a sequence of interest located either between the inverted repeat (IR) or 3' to the inverted repeat, and (3) a primer binding site (PBS) for the reverse transcriptase that is located 3' to the inverted repeat.
The vector system also preferably includes the functions and signaling instructions for transcription of these elements *in vivo* and the functions and signaling instructions for translation of the reverse transcriptase (RT) gene. Additional elements that may be included in the set of genetic elements of the present invention include a restriction endonuclease (RE) gene, which may be included for a purpose described below, and a sequence having enzymatic activity when the linearized ss-DNA folds into the secondary configuration which confers enzymatic activity upon the sequence. The enzymatic sequence is preferably located within a sequence of interest, regardless of whether the sequence of interest is located between the inverted tandem repeats or between the 3' aspect of the inverted repeat and the PBS.

In a first embodiment of the vector system described herein, the vector comprises a two plasmid system, and the first plasmid includes the RNA-dependent DNA polymerase (reverse transcriptase) gene and an RNAse H gene, linked with a histidine-proline linker to a restriction endonuclease gene. The plasmid into which these genes were inserted contains the necessary transcriptional and translational control elements for these genes along with polyadenylation tailing sequences. This plasmid is referred to herein as the "A" plasmid (pssDNA-Express-A as shown in Fig. 4 in one of the preferred embodiments described herein). A second, "B" plasmid was constructed which, in the embodiment described herein, includes the cassette comprised of three of the above-listed elements, namely, a primer binding sequence (PBS) matched to the reverse transcriptase, a sequence of interest, and an inverted tandem repeat. In two embodiments described herein (pMN-new-link and pssDNA-Express-4B, the latter being shown in Fig. 7B), the sequence of interest is positioned either between the inverted tandem repeats or in a 5' position (with respect to the mRNA transcript) to the primer binding sequence (PBS), the PBS being located at the most 3' aspect of the mRNA transcript. In other words, the sequence of interest is located (1) inbetween the inverted repeats, (2) between the inverted repeats and the PBS, and/or (3) both between the inverted repeats and inbetween the inverted repeats and the PBS**.** Like plasmid A, plasmid B also includes a combination of transcriptional control elements. However, in at least one preferred embodiment described herein, the B plasmid does not include (or require) translational control elements since no protein product is produced from this construct.

In another embodiment described herein, the vector system of the present invention comprises a single plasmid, designated as plasmid C, in which all of the elements of the set of genetic elements listed above were incorporated with the exception of the RE gene, which was not included for reasons to be made clear in the following discussion. Additionally, the components of the cassette included in the B plasmid of the two plasmid system described above, e.g., the primer binding sequence (PBS), sequence of interest, and inverted tandem repeat, reside in the untranslated 3' portion of the reverse transcriptase polyprotein in the C plasmid. In other words, when the RT-RNAse H component of the C plasmid is transcribed under control of an appropriate promoter (in the preferred embodiments described herein, the RSV promoter was utilized), the resulting mRNA transcript contains the coding region for the reverse transcriptase-RNAse H polyprotein and, at the end of translation at the stop signals, the additional mRNA transcript contains (3' to this translated protein) the elements from the B plasmid with further 3' downstream signaling events for polyadenylation signals, which remain intact from the RT-RNAse H component.

The particular single plasmid vector system described herein (pssDNA-Express-C as shown in Fig. 9) does not contain the restriction endonuclease gene, and therefore does not include the ability to digest the stem of the stem-loop intermediate formed by the inverted repeats. Consequently, the sequence of interest (including the DNA enzyme) is inserted into the C plasmid only in a 3' position to the inverted repeats and unwanted vector sequences are removed by premature truncation of the ss-cDNA product as the transcript encounters the relatively stable stem and is unable to continue transcribing the ss-cDNA from the mRNA transcript. More specifically, as will be made apparent in the following description, each of the sequences of interest was inserted only within the Bam H I-Pac I restriction sites of the pssDNA-Express-C plasmid.

As will also be apparent from the following description of the B and C plasmids, the plasmids include cloning sites for insertion of the sequence of interest. Both Not 1 sites (located between the inverted repeats) and Pac I/BamH I (3' to the inverted repeats, e.g., between the inverted repeats and the PBS) sites are provided in the preferred embodiment of the B plasmid described herein. The preferred C plasmid described herein includes only the Pac I/BamH I sites for this purpose. However, those skilled in the art who have the benefit of this disclosure will recognize that these particular cloning sites were chosen for the particular systems described herein and that other cloning sites may be equally useful for this same purpose. The particular A plasmid comprising the two plasmid vector system described herein was not intended to include the sequence of interest, but those skilled in the art having the benefit of this disclosure will recognize that, if a two plasmid vector system is to be used, the elements of the set of genetic elements of the present invention, and particularly the sequence of interest, may be inserted into either plasmid as may be convenient.

The nucleic acid sequence that is referred to herein as a cassette provides the template for synthesis of ss-cDNA in target cells. It is this element which includes the sequence of interest, inverted tandem repeat, and primer binding site. As is the case for the other elements of the set of genetic elements of the present invention, this genetic element is preferably regulated by an appropriate wide spectrum or tissue-specific promoter/enhancer, such as the CMV promoter, or combination of promoters/enhancers, located upstream of the genetic element. Also as is the case for the other genetic elements, the promoter/enhancer can either be constitutive or inducible promoter. Those skilled in the art who have the benefit of this disclosure will recognize that a number of other eukaryotic promoters may be used to advantage to control expression of the sequence of interest including SV-40, RSV (non-cell type specific) or tissue specific glial fibulary acidic protein (GFAP).

As shown in Fig. 1, for expression in eukaryotic cells, the cassette also includes a downstream polyadenylation signal sequence so that the mRNA produced by the sequence of interest has a poly(A) tail. The primer-binding site (PBS) for initiation of priming for cDNA synthesis is located between the 3' inverted tandem repeat and the polyadenylation signal. The PBS is a sequence which is complementary to a transfer RNA (tRNA) which is resident within the eukaryotic target cell. In the case of the mouse Maloney reverse transcriptase described herein as being utilized in conjunction with the present invention, the PBS takes advantage of the proline tRNA. The PBS utilized in connection with the presently preferred embodiment of the invention that is described herein was taken from the actual 18 nucleotide sequence region of mouse Moloney virus. *See* 293 Nature 81. In the case of the reverse transcriptase gene from human immunodeficiency virus that was also tested as noted above, the PBS used was taken from the nucleotide sequence of HIV. Y. Li, *et al.,* 66 J. Virology 6587-6600 (1992). In short, any PBS that is matched to the reverse transcriptase which is utilized in connection with the method of the present invention is utilized for this purpose.

The cassette of the present invention also comprises a pair of inverted tandem repeats. After digestion of the mRNA from the mRNA-cDNA heteroduplex by RNAse H and the release of the ss-cDNA, the inverted repeats cause the ss-cDNA to fold back upon itself to form the stem of a stem-loop structure, the stem structure being comprised of double stranded, anti-parallel DNA. The ss-cDNA which is produced is transcribed with the encoded 5' and 3' regions flanking the stem (made up of the inverted repeat) and a loop containing the sequence of interest. One or more functional genetic elements may be designed into the double stranded portion, i.e., the inverted repeat, that forms the stem of the stem-loop intermediate. In the examples described herein, the functional genetic element is one or more restriction endonuclease site(s) which is cut by the restriction endonuclease produced from the above-described restriction endonuclease gene (in the case of those plasmids which include an RE gene), but those skilled in the art will recognize that the inverted repeat can be designed to include other functional genetic elements such as a specific enzyme recognition sequence (other than a restriction endonuclease site), eukaryotic and/or prokaryotic receptor recognition sites, promoter or promoter/enhancer sites for driving expression of a target sequence (that may or may not be likewise designed into the stem) in isolated *cis*-oriented fashion. In the event the functional genetic element comprises a specific enzyme recognition sequence such as a restriction endonuclease site, the stem is then cut (also termed digested or cleaved) by any of the many restriction endonuclease enzymes which recognize the cut site designed into the stem (note that the endonuclease recognition site may be designed into the stem even though the RE gene is not included in the vector system of the present invention) to release the ss-cDNA loop. The loop portion of the ss-cDNA, which does not form any apparent duplex DNA, is immune to restriction endonuclease activity since restriction endonucleases recognize only double stranded DNA as a target substrate.

It will be recognized by those skilled in the art that the restriction endonuclease site(s) need not be designed into the inverted repeats which form the stem of the stem-loop intermediate if the second aspect of the present invention is being utilized. In other words, if it is desired to produce ssDNA from a second sequence of interest located between the primer binding site and the inverted repeats, with transcription of the cassette to terminate at the stem formed by the inverted repeats, there is no need for a restriction endonuclease site in the stem. Another option is to design the inverted repeats to contain eukaryotic, prokaryotic, or viral protein DNA binding sites, which can act to competitively titer out selected cellular proteins. Combinations of restriction sites or other sequence specific elements may be included in the inverted tandem repeats depending on the base pair composition chosen for the construction of inverted repeats such that linear or precisely cut stem-loop intermediate forms of ss-DNA are produced. It is generally preferred to use synthetically constructed functional genetic elements in the inverted tandem repeat since (if the functional genetic element is a restriction endonuclease site) it is unlikely that a naturally occurring inverted repeat would have the properly aligned restriction sites.

As noted above, the cassette which comprises one of the elements of the set of genetic elements of the present invention optionally also includes a DNA sequence with catalytic activity. Because of the inclusion of the so-called "DNA enzyme" in the cassette, and specifically, it is contemplated that the DNA enzyme is located within the sequence of interest, the present invention is used to particular advantage when the sequence of interest serves as the template for synthesis of an inhibitory nucleic acid that is an antisense sequence. For that reason, the examples set out herein describe production of four antisense sequences of interest as set out in Fig. 6, each including a sequence having enzymatic activity against mRNA, including C-raf kinase, h-ras antisense sequence, antisense sequence to pleiotrophin angiogenic growth factor, and the tat region of simian immunodeficiency virus (SIV). Those skilled in the art will recognize, however, that the present invention is not limited just to antisense sequences and that the inhibitory nucleic acid sequence could also be any of the other types of inhibitory nucleic acid sequences described above.

The nucleic acid sequence having enzymatic activity which comprises the cassette of the present invention can be any such enzymatic sequence. The sequence having the desired catalytic activity is inserted into the cassette in either or both of the two locations, e.g., (a) between the inverted repeats and inside the sequence of interest or (b) inside the second sequence of interest that is located 3' to the inverted repeats and 5' to the PBS. Either way, the resulting aptamer is specific for the target of the sequence of interest and is therefore used to target other DNA sequences, mRNA sequences, and any other suitable substrate, to inhibit or change DNA or mRNA splicing mechanisms, or even to directly alter the cellular genome in a specific manner.

Nucleic acid sequences with enzymatic activity that are appropriate for use in connection with the present invention include, but are not limited to,
sequences having RNAse activity such as the so-called "10-23" and "8-17 enzymes" (Santoro, S.W. and G.F. Joyce, *supra* (1997)) and other metal-dependent RNAses (Breaker, R.R. and G.F. Joyce, 1 Biol. Chem. 223-229 (1994); Breaker, R.R. and G.F. Joyce, 2 Biol. Chem. 655-660 (1995)) and histidine-dependent RNAse (Roth, A. and R.R. Breaker, 95 Proc. Natl Acad. Sci. USA 6027-6031 (1998));
sequences having DNAse activity such as copper-dependent DNAse (Carmi, N., *et* al., 3 Chem. Biol. 1039-1046 (1996); Carmi, N., *et al.,* 95 Proc. Natl Acad. Ser. USA 2233-2237 (1998); Sen, D. and C.R. Geyer, 2 Curr. Opin. Chem. Biol. 680-687 (1998)) and the DNAses which required divalent metal ions as cofactors or hydrolyzed the substrate independently of divalent metal ions as reported in Faulhammer, D. and M. Famulok (269 J. Molec. Bio. 18-203 (1997));
sequences with DNA ligase activity such as copper-dependent DNAse (Breaker, R.R., 97 Chem. Rev. 371-390 (1997)) and zinc-dependent E47 ligase (Cuenoud, B. and J.W. Szostak, 375 Nature 611-613 (1995));
sequences with DNA kinase acitivity such as calcium-dependent DNA kinase (Li, Y. and R.R. Breaker, 96 Proc. Natl. Acad. Sci. USA 2746-2751 (1999)); and
sequences with RNA kinase acitivity such as calcium-dependent DNA kinase (Li, Y., *supra* (1999)).
The particular nucleic acid sequence having enzymatic activity utilized in the examples described herein is the "10-23 enzyme" (Santoro, S.W. and G.F. Joyce, *supra* (1997)), and it is this enzymatic sequence which is represented schematically in Figs. 3A and 3B. However, those skilled in the art will recognize from this disclosure that any of the sequences reported in the above-listed literature will function for the intended purpose when inserted into the cassette of the present invention.

Regarding the RNA-dependent DNA polymerase, or reverse transcriptase (RT) gene which also comprises one element of the set of genetic elements of the present invention, as noted above, the reverse transcriptase/RNAse H gene from Moloney murine leukemia virus was used to advantage in the examples described herein. The reverse transcriptase/RNAse H gene from the human immunodeficiency virus (HIV) has also been tested. Many other retroviral reverse transcriptase/RNAse H genes may be used to advantage in connection with the present invention including those from retroviruses such as SIV, strains of hepatitis B, hepatitis C, bacterial retron elements, and retrons isolated from various yeast and bacterial species, it being preferred that the reverse transcriptase/RNase H gene be a reverse transcriptase/RNase H gene that is regulated by an appropriate upstream promoter/enhancer such as the cytomegalovirus (CMV) or Rouse sarcoma virus (RSV) promoter for expression in eukaryotic cells.

As found in nature, these RNA-dependent DNA polymerases usually have an associated RNase H component enzyme within the same coding transcript. However, the present invention does not require the naturally-occurring RNase H gene for a particular reverse transcriptase. In other words, those skilled in the art will recognize from this disclosure that various combinations of reverse transcriptase and RNase H genes can be spliced together for use in connection with the present invention to fulfill this function and that modifications and/or hybrid versions of these two enzyme systems are available and/or known to those skilled in the art which will function in the intended manner. Those skilled in the art will also recognize that the target cell may itself have sufficient endogenous RNase H to fulfill this function. Similarly, those skilled in the art will recognize that the target cell may itself have sufficient endogenous reverse transcriptase activity from, for instance, prior retroviral infection, to fulfill this function.

The reverse transcriptase/RNase H gene also preferably includes a downstream polyadenylation signal sequence so that the mRNA produced from the reverse transcriptase/RNase H gene includes a 3' poly(A) tail for mRNA stability. As known to those skilled in the art, multiple poly(A) tails are available and are routinely used for production of expressed eukaryotic genes.

The reverse transcriptase produced in the cell synthesizes a complementary DNA (cDNA) using as the template the genetic element including the sequence of interest described below. The RNase H activity of the reverse transcriptase, along with endogenous RNAse activity within the cell, degrades the mRNA template component of the RNA/cDNA hybrid to produce ss-DNA *in vivo.*

The gene encoding the restriction endonuclease (used only in the two plasmid system, and not even a required component of that system) may be any of several genes which encode for restriction endonucleases, and preferably those that are controlled by one or more constitutive or inducible wide spectrum and/or tissue-specific promoters/enhancers as discussed below. The particular restriction endonucleases utilized herein were Mbo II and Fok I, but those skilled in the art who have the benefit of this disclosure will recognize that any restriction endonuclease (type I, II, IIS, or III) site may be included in the inverted tandem repeat. These enzymes "clip," or digest, the stem of the stem-loop intermediate to linearize the sequence of interest as single-stranded DNA.

Although expression of the RE gene may be regulated by an appropriate constitutive or inducible promoter/enhancer located upstream from the restriction endonuclease gene such as the CMV or RSV promoter for expression in human cells, in plasmid pssDNA-Express-A described herein, the RE gene (Mbo II) is linked to the RT-RNAse H polypeptide. The RE gene also preferably includes a downstream polyadenylation signal sequence so that the mRNA transcript from the restriction endonuclease gene will have a 3' poly(A) tail.

Those skilled in the art who have the benefit of this disclosure will also recognize that a number of tissue-specific or wide spectrum promoters/enhancers, or combinations of promoters/enhancers other than those listed above may also be used to advantage to regulate the reverse transcriptase/RNAse H gene, the RE gene (if utilized), and the sequence of interest. Although a list of all available promoters/enhancers is not needed to exemplify the invention, as noted above, the promoters/enhancers may be constitutive or inducible and may include the CMV or RSV (non-cell type specific) or GFAP (tissue specific) promoters/enhancers and many other viral or mammalian promoters. Representative promoters/enhancers which are appropriate for use in connection with the elements of the present invention may include, but are not limited to, HSVtk (S.L. McKnight, *et al.,* 217 Science 316 (1982)), human β-globulin promoter (R. Breathnach, *et al.,* 50 Ann. Rev. of Biochem. 349 (1981)), β-actin (T. Kawamoto, *et al.,* 8 Mol. Cell Biol. 267 (1988)), rat growth hormone (P.R. Larsen, *et al.,* 83 Proc. Natl. Acad. Sci. U.S.A. 8283 (1986)), MMTV (A.L. Huang, *et al.,* 27 Cell 245 (1981)), adenovirus 5 E2 (M.J. Imperiale, *et al.,* 4 Mol. Cell. Biol. 875 (1984)), SV40 (P. Angel, *et al.,* 49 Cell 729 (1987)), a-2-macroglobulin (D. Kunz, *et al.,* 17 Nucl. Acids Res. 1121 (1989)), MHC class I gene H-2kb (M.A. Blanar, *et al.,* 8 EMBO J. 1139 (1989)), and thyroid stimulating hormone (V.K. Chatterjee, *et al.,* 86 Proc. Natl. Acad. Sci. U.S.A. 9114 (1989)).

When the elements comprising the set of genetic elements of the present invention are incorporated into a vector, it is preferred that the vector contain other specialized genetic elements to facilitate the identification of cells that carry the vector and/or to increase the level of expression of the cassette. The specialized genetic elements include selectable marker genes so that the vector can be transformed and amplified in a prokaryotic system. For example, the most commonly used selectable markers are genes that confer to the bacteria (e.g., *E. coli*) resistance to antibiotics such as ampicillin, chloramphenicol, kanamycin (neomycin), or tetracycline. It is also preferred that the vector contain specialized genetic elements for subsequent transfection, identification and expression in a eukaryotic system. For expression in eukaryotic cells, multiple selection strategies (e.g., Chinese Hamster Ovarian: CHO) may be used that confer to the cell resistance to an antibiotic or other drug or alter the phenotype of the cell such as morphological changes, loss of contact inhibition, or increased growth rate. Selectable markers used in eukaryotic systems include, but are not limited to, resistance markers for Zeocin, resistance to G418, resistance to aminoglycoside antibiotics, or phenotypic selection markers such *β-gal* or green fluorescence protein.

Incorporation of these components into an appropriate vector allows two convenient methods for removing predetermined vector sequences after the production of ssDNA. In the first method, the loop portion of the ssDNA stem-loop intermediate that is produced is comprised of the nucleotide sequence of interest and, after digestion with the restriction endonuclease, the loop is released as linearized, single-stranded cDNA without flanking sequences. In the second method in which a second sequence of interest is included in the cassette 3' to the inverted tandem repeat, reverse transcription is terminated at the stem of the stem-loop structure such that the resulting ssDNA is produced without flanking sequences. If it is desired to produce ssDNA utilizing the second method, the cassette is designed with an inverted repeat which forms a stem that is more stable than the stem of the stem-loop intermediate from which the ssDNA is produced by digestion of the stem in accordance with the first aspect of the present invention. By designing the cassette with an inverted repeat that forms a stem that is easily denatured in accordance with the first aspect of the invention, reverse transcription proceeds right on through the second sequence of interest (if it is even designed into the cassette) to the sequence of interest located between the inverted repeat. A stem which is intermediate in stability allows production of both the first and second sequences of interest.

This "premature termination" of the reverse transcriptase cDNA transcript at the 3' aspect of the stem structure was discovered when a vector with a stem including 29 base pairs was used in *in vitro* experiments, the additional base pairs included in the stem providing the additional stability which causes production of both the first and second sequences of interest. The design of a stem-loop intermediate having a stem of the desired stability to obtain either production of the first (between inverted repeat) or second (between the 3' aspect of the inverted repeat and the PBS) is within the skill of those skilled in the art who have the benefit of this disclosure. In brief, factors such as the length of the inverted repeat (e.g., the number of nucleotides comprising the repeat) and the identity of the bases comprising the repeat can be manipulated (using, for instance, the *in vitro* system described herein) to obtain a stem having as little or as much stability as may be desired to obtain production of the first or second sequence of interest or a mix of both sequences.

It will also be evident to those skilled in the art from this description that the intact stem-loop ss-cDNA structure can function similarly in many applications as the linearized ss-cDNA form. Consequently, the cassette is also used to advantage without the restriction endonuclease gene and associated regulatory elements and/or with a sequence of interest which lacks the corresponding restriction endonuclease site.

It will also be evident to those skilled in the art from this description of the preferred embodiments of the present invention that a cassette can be made which encodes a ss-cDNA that has a "trimmed" stem-loop structure. The restriction endonuclease sites encoded in the inverted repeats flanking the sequence of interest are designed such that the stem portion (after duplex formation) is digested with the corresponding restriction endonuclease so as to cut the dsDNA comprising the stem in a way that removes a portion of the stem and the associated flanking sequences yet leaves sufficient duplex DNA that the transcript retains the above-described stem-loop structure. Such a ss-cDNA structure may be more resistant to intracellular nucleases by retaining the "ends" of a ssDNA in double stranded form.

It will also be evident to those skilled in the art that the stem (duplex DNA) can be designed to contain a predetermined sequence (or sequences), i.e., aptamers, that are recognized and bound by specific DNA-binding proteins. Among other uses, such stem structure is used in the cell as a competitor to titer out a selected protein(s) that regulates specific gene function. For example, a ss-cDNA stem-loop is produced in accordance with the present invention in a cell that contains a binding site for a selected positive transcription factor such as adenovirus E1a. Adenovirus E1a, like other oncogenes, modulates expression of several adenoviral and cellular genes by affecting the activity of cell-encoded transcription factors, resulting in the changing of normal cells to transformed cells. Jones, *et al.,* 2 Genes Dev. 267-281 (1988). The duplex stem of the stem-loop intermediate produced in accordance with the present invention is therefore designed to function to "bind up" this transcription factor, preventing the protein from binding a promoter, and thus inhibiting expression of the particular deleterious gene. To those skilled in the art, it will be clear that the duplex stem structure may optionally contain multiple binding sites, for example, sites which are recognized by various transcription factors that actively regulate expression of particular gene. For example, adenovirus E1a has been found to repress transcription of the collagenase gene via the phorbol ester-responsive element, a promoter element responsible for the induction of transcription by 12-O-tetradecanolyphorbol 13-acetate (TPA), by a number of other mitogens, and by the *ras, mos, src,* and *trk* oncogenes. The mechanism involves inhibition of the function of the transcription factor family AP-1. Offringa, *et al.*, 62 Cell 527-538 (1990). Any desired nucleotide sequence can be inserted into the genetic element which encodes the "loop" portion of the stem-loop intermediate to ultimately carry out a desired inhibitory function, e.g., antisense binding, down regulation of a gene, and so on as herein described.

In another aspect which will be recognized by those skilled in the art, the present invention is used to construct complex secondary ssDNA structures which confer biologic reactions on the cDNA transcript based on conformational secondary structure folding. Such secondary structure can be engineered to serve any of several functions. For instance, the sequence of interest may include (but is not limited to) a sequence which is incorporated into the loop portion of the single-stranded cDNA transcript which forms so-called "clover leaf" or "crucible" like structures such as those found in the long terminal repeats of adeno-associated virus or in retrotransposons. Under correct circumstances, such structure is integrated in site-specific manner into the host genome.

Because the set of genetic elements of the present invention is adaptable for incorporation into multiple commercially available delivery vectors for mammalian and human therapeutic purposes, multiple delivery routes are feasible depending upon the vector chosen for a particular target cell. For example, viral vectors are presently the most frequently used means for transforming the patient's cells and introducing DNA into the genome. In an indirect method, viral vectors carrying new genetic information are used to infect target cells removed from the body and the infected cells are then re-implanted (i.e., *ex vivo*). Direct *in vivo* gene transfer into postnatal animals has been reported for formulations of DNA encapsulated in liposomes and DNA entrapped in proteoliposomes containing viral envelope receptor proteins. Nicolau, *et al.,* 80 Proc. Natl. Acad Sci USA 1068-1072 (1983); Kaneda, *et al.,* 243 Science 375-378 (1989); Mannino, *et al.,* 6 Biotechniques 682-690 (1988). Positive results have also been described with calcium phosphate co-precipitated DNA. Benvenisty and Reshef, 83 Proc. Natl. Acad. Sci. USA 9551-9555 (1986). Other systems that are used to advantage include intravenous, intramuscular, and subcutaneous injection, as well as direct intra-tumoral and intra-cavitary injection. The set of genetic elements, when incorporated into the vector of choice, is also advantageously administered through topical, transmucosal, rectal, oral, or inhalation-type methods of delivery.

The vector including the set of genetic elements of the present invention is advantageously employed to deliver anti-sense, triplex, or any other inhibitory nucleic acid or single-stranded nucleotide sequence of interest, using known digestion and ligation techniques to splice the particular sequence of interest into the cassette (between inverted tandem repeat or between PBS and inverted tandem repeat). Those skilled in the art who have the benefit of this disclosure will also recognize that the above-described signals used for expression within eukaryotic cells may be modified in ways known in the art depending upon the particular sequence of interest. For instance, a likely change is to change the promoter so as to confer advantageous expression characteristics on the cassette in the system in which it is desired to express the sequence of interest. There are so many possible promoters and other signals, and they are so dependent on the particular target cell for which the sequence of interest has been selected, that it is impossible to list all the potential enhancers, inducible and constitutive promoter systems, and/or poly(A) tailing systems which may be preferred for a particular target cell and sequence of interest.

In another embodiment, the present invention takes the form of a kit comprised of a plasmid having the above-described RNA-dependent DNA polymerase and restriction endonuclease genes cloned therein as well as a multiple cloning site (MCS) into which the user of the kit inserts a particular sequence(s) of interest. The cloning site into which the sequence(s) of interest is inserted is located between the above-described inverted tandem repeats, e.g., upstream from the genetic element encoding the primer binding site. The resulting plasmid is then purified from the cell culture in which it is maintained, lyophilized or otherwise preserved for packaging and shipping, and sent to the user. The kit preferably also includes the restriction endonuclease for the cloning site into which the sequence of interest is to be cloned, the ligases and other enzymes, along with suitable buffers, for ligating the sequence of interest into the plasmid, and a map of the plasmid and/or other instruction for the user.

In the embodiments described herein, the sequences of interest are delivered to a host cell either by co-transfection of the cells with two plasmids, designated A and B, each plasmid being designed and constructed to include one or more of the genetic elements listed above, or a single "C" plasmid. To summarize, in the two plasmid system, the B plasmid encodes the cassette including the sequence of interest, nested within flanking sequences that include the inverted repeat, and the primary binding site that provides the post-transcriptional processing signals that mediate the conversion of the mRNA into single-stranded DNA. The B plasmid also includes the second sequence of interest when this second aspect of the invention is utilized (as set out above, when the RE gene is omitted from the construct of the present invention, for instance in the single "C" plasmid described herein, it is this second sequence of interest that encodes the particular inhibitory nucleic acid having the desired activity). Activities required for processing the primary gene product of the B plasmid into single-stranded DNA, with the removal of vector sequences and processing signals, specifically the reverse transcriptase/RNAse H, and restriction endonuclease, are expressed from the A plasmid. The single-stranded DNA sequence that is released by interaction of the transcriptional products of these components *in vivo* is free to bind intracellular targets such as mRNA species and DNA promoters in antisense and triplex strategies.

As noted above, as described herein, the B plasmid includes cloning sites (*Not* I sites were utilized in one embodiment of the B plasmid described herein) between which any DNA sequence of interest is placed (as noted above, in the examples described herein, the sequences include a "stuffer," or test, sequence, telomeric repeats, h-ras, c-raf kinase, a region encoding the angiogenic growth factor pleiotrophin, and the region encoding tat from SIV). Flanking the cloning sites are signals directing the processing of the primary mRNA transcript, produced from a promoter (a CMV promoter was utilized in the preferred B plasmid described herein), into the desired single-stranded inhibitory nucleic acid. After cloning of the desired sequence of interest into the B plasmid, the A and B plasmids are co-transfected into a cell line of choice for constitutive expression of ssDNA. Similarly, in the single ("C") plasmid system described herein, the sequence of interest is cloned into that plasmid and transfected into the cell line for further processing. Regardless of the distribution of the elements of the above-described set of genetic elements between two (or even more) plasmids, or if the elements are all contained in a single plasmid, this processing proceeds in three steps following transcription of the single-stranded DNA region (i.e., sequence of interest, inverted repeats, and PBS):
(1) reverse transcription of the B or C plasmid RNA transcript by a reverse transcriptase, which in the preferred embodiment described herein is reverse transcriptase expressed by the A or C plasmid (in the preferred embodiment described herein, the reverse transcriptase is Moloney mouse leukemia virus (MoMuLV) reverse transcriptase), proceeding from the primer binding site lying 3' to the sequence of interest (including the sequence with enzymatic activity), inverted repeats, and primer binding site as shown in Fig. 1;
(2) RNAse H digestion of the resulting heteroduplex, either by the RNAse H activity of the reverse transcriptase polyprotein or by endogenous RNAse H activity, to release the single-stranded DNA precursor from its RNA complement; and
(3) Removal of flanking sequences by either restriction endonuclease digestion of the stem of a stem-loop intermediate formed upon Watson-Crick base pairing of the bases comprising the inverted repeat or by premature termination of the cDNA transcript by formation of the stem-loop secondary structure by the self-complementary inverted tandem repeats.
Those skilled in the art will recognize from this disclosure that the particular cloning sites flanking the sequence of interest, the particular reverse transcriptase, restriction endonuclease (if utilized), promoter, primer binding site, and all the other elements of the set of genetic elements of the present invention are chosen depending upon the particular sequence of interest and/or system in which the ssDNA is to be expressed.

Except where otherwise indicated, standard techniques as described by Seabrook, *et al.* (1989) (J. Seabrook, *et al.,* Molecular Cloning: A Laboratory Manual (2nd Ed.), Cold Spring Harbor Press (1989), hereinafter referred to as "Maniatis, *et al.* (1989)") and Ausubel, *et al.* (1987) (F.M. Ausubel, *et al.,* Current Protocols in Molecular Biology, New York: John Wiley & Sons (1987)), were utilized in the examples set out below.

The plasmid pcDNA3.1/Zeo(+) was purchased from Invitrogen Corp. (Carlsbad, CA) and plasmid pBK-RSV from Statagene (La Jolla, CA). Oligodeoxynucleotides (ODN) were synthesized by Midland Certified Reagent Co. (Midland, TX). Polymerase chain reactions (PCR) were carried out using Taq DNA polymerase purchased from Boehringer Mannheim Corp. (Indianapolis, IN) in a Robo-gradient thermal cycler (Stratagene (La Jolla, CA). Restriction endonucleases and T4 DNA ligase were obtained from Boehringer Mannheim Corp. (Indianapolis, IN). The ODNs used are listed in the attached Sequence Listing.

### EXAMPLE 1. In vitro Synthesis of ss-DNA

Four synthetic, single-stranded ODNs (129, 121, 111, and 103 bases in length, Sequence ID Nos. 4, 5, 15, and 16, respectively) were designed (see the Sequence Listing in Table 1) to contain an inverted tandem repeat structure with a so-called "stuffer" region of random nucleotide sequence composition inserted as a sequence of interest between the inverted tandem repeat. The inverted tandem repeat was designed in such a way (Fig. 2) as to have a pair of restriction endonuclease recognition cut sites within the repeats. The designed cut sites for Not I and Fok I (type II and type IIS restriction endonuclease recognition cuts, respectively) were the 111 (Seq. ID 15) and 102 (Seq. ID 16) base length oligonucleotides and the designed restriction endonuclease cut sites for Not I and Mbo II were the oligonucleotides of length 129 (Seq. ID 4) and 121 (Seq. ID 5) bases. In addition, a tRNA primer binding sequence (PBS) for recognition by primer for reverse transcriptase was designed into the oligonucleotides. The PBS was located 3' downstream from the inverted tandem repeat.

For each of the synthetic oligonucleotides, 1 µl (5 µg/µl in water) aliquots were added to four separate tubes and heated to 70° for 5 minutes and allowed to self anneal over 15 minutes at room temperature. This process allows optimal hybridization for the formation of a stem-loop structure; the loop portion, which does not have stretches of complementary sequence, should not undergo significant self-annealing and remain predominantly single-stranded. Standard restriction endonuclease digests were then carried out with Not I, Fok I, Mbo II (appropriate for each oligonucleotide for which a restriction endonuclease cut site was present) and EcoR I (as a negative control) with 10 units of enzyme in a total reaction volume of 15 µl and appropriate reaction buffers. The digests were confirmed by electrophoretic gel analysis.

The results of this experiment showed that a synthetic ss-cDNA with inverted repeats formed duplex DNA. The duplex DNA, presumably the stem of a stem-loop structure, formed specific and recognizable restriction endonuclease sites from the sequences in the inverted tandem repeats, which were designed so as to form Not I and Fok I restriction endonuclease sites by Watson-Crick base pairing of the bases comprising the inverted tandem repeats. When the ss-cDNA was incubated with Not I and Fok I, the DNA was digested. When incubated with EcoR I (negative control), the same DNA was not digested.

### EXAMPLE 2. In vitro Formation of ss-DNA From Cassette Transcripts

Two test plasmids were constructed to conduct this experiment. pcDNA3.1/Zeo(+) was digested with Nhe I and Apa I under standard conditions. Two sets (A and B) of 5' phosphorylated oligonucleotides, which were designed to be complementary with each other (see attached sequence listings) were allowed to hybridize. Hybridization was performed by heating the complementary oligonucleotides at 95°C and then allowing them to cool to room temperature for 15 minutes. The resultant duplex oligonucleotide linkers with appropriate cohesive ends were ligated under standard conditions to the previously prepared pcDNA3.1/Zeo(+) vector.

The selection of positive clones on ampicillin plates was performed after transformation into competent XL1-Blue MRF' cells (Stratagene) as described by Maniatis, *et al.* (1989) and the accompanying instruction. After positive clones were picked, plasmid DNA was isolated using a commercially available plasmid isolation kit (Quiagen, Inc., Sant Caita, CA). Confirmation of DNA ligation was carried out by DNA sequencing.

Positive circular test plasmids were linearized by digestion with Pme I, and standard reverse transcription reactions were carried out as follows. To each tube was added 0.1 µg of linearized plasmid DNA, 25 units of T7 polymerase (i.e., a DNA-dependent RNA polymerase), 2.5 mM rNTP's (ribonucleotide triphosphates rUTP, rCTP, rGTP, rATP) and appropriate buffer. The reaction tube was incubated at 37°C for 90 minutes. After incubation, 10 units of DNAase was added and incubated for 15 minutes at 37°C. The reaction was terminated by incubation at 70°C for 10 minutes.

Standard cDNA synthesis reactions were carried out using the above reactions. To a fresh tube was added 5 µl of the above reverse transcriptase reaction, 0.2 µg primer (see sequence listing) complementary to the PBS region (downstream from inverted tandem repeats). To this mixture was added 25 units of reverse transcriptase from Moloney murine leukemia virus, 2.5 mM dNTPs (deoxyribonucleotide triphosphates dTTP, dCTP, dGTP, dATP), and the appropriate reaction buffer. The reaction mixtures were incubated at 37°C for 1.5 hours. After the incubation period, 100 units of RNase H was added and the tubes were incubated at 37°C for 15 minutes. The reaction tubes were incubated at 70°C and allowed to anneal by cooling to room temperature over 15 minutes. The mixture was divided equally into four tubes, in which either 10 units of restriction enzyme Not I, Fok I, Mbo II, or EcoR I was added along with the appropriate reaction buffer. The tubes were incubated at 37°C for 1 hour. Replicate reactions were treated with S1 nuclease (specific for ssDNA). The DNA from the above reactions was resolved by gel electrophoresis.

This experiment demonstrated the *in vitro* production of ssDNA by the sequential enzymatic activities of T7 RNA polymerase, reverse transcriptase, and RNase H. The linker contained inverted tandem repeats with the restriction sites Not I, Fok I, and Mbo II; a "stuffer" region (which comprises the loop of the stem-loop intermediate), and a tRNA PBS. The plasmid contained the T7 promoter immediately upstream of the linker region. Agarose gel electrophoresis analysis showed the stepwise production of (1) an mRNA intermediate of predicted length, (2) a cDNA of predicted length, and (3) the subsequent digestion of the ssDNA by the corresponding restriction endonucleases Not I, Fok I, and Mbo II. A negative control in which EcoRI was used did not digest the ss-cDNA.

### EXAMPLE 3. In vivo Synthesis of ss-cDNA in Eukaryotic Cells

The following *in vivo* experiments were designed to test the plasmids made in Example 2 that contained different "stuffer" regions which were inserted between the inverted tandem repeats. The tissue culture cells used in these experiments encoded and endogenously expressed eukaryotic DNA-dependent RNA polymerase II, which utilizes the RSV promoter located upstream of the cloned genetic element that is the linker/stuffer segment described in Example 1, above. It will be recognized by those skilled in the art who have the benefit of this disclosure that any eukaryotic promoter can be used for this purpose. It is further recognized by such persons that a vector-encoded DNA-dependent RNA polymerase will also function in this capacity.

**Plasmid constructs.** The ODNs were allowed to hybridize in 1 µl (5 µg/µl in water) in four separate tubes which were incubated at 70°C for 5 min and allowed to hybridize for 15 min at room temperature. Standard restriction endonuclease digests were carried out (EcoR I used as a negative control) with 10 units of enzyme in a total reaction volume of 15 µl and appropriate reaction buffers. DNA fragments were resolved in and isolated from agarose gels. The selection of positive clones on ampicillin plates was performed after transformation into competent XL1-Blue MRF cells (Stratagene) as described by Maniatis, *et al.* (1989). After positive clones were selected, plasmid DNA was isolated using the above-described Quiagen plasmid isolation kit.

The construction of three expression plasmids is described. The first "B" plasmid was derived from pcDNA3.1/Zeo(+) (Fig. 7A) by digesting with restriction endonucleases Nhe I and Apa I, which are located in the multiple cloning site (MCS). The double-stranded oligodeoxynucleotide having compatible *Nhe* I and *Apa* I ends which is formed by annealing the synthetic, single stranded oligodeoxynucleotides Seq. ID 4/ODN-PMMV(+) and Seq. ID 5/ODN-PMMV(-) was ligated into the digested pcDNA3.1/Zeo(+) to give pPMMV. This insert contains the Moloney Mouse leukemia virus reverse transcriptase (MoMuLV-RT) promoter region/primer binding site (PBS sequence). It also contains two *Not* I sites, unique to pMMV, and an *Mbo II* site. In this plasmid, and the plasmids derived from this construct, the strands designated (+) are positioned to be transcribed into RNA from the cytomegalovirus promoter of pcDNA3.1/Zeo(+). The B plasmid, pssDNA-Express-B, containing sites for convenient insertion of a sequence of interest to be expressed *in vivo* as ssDNA, was obtained by ligating the double-stranded sequence formed by annealing ODN-XB(+) and ODN-XB(-), which has *Not* I compatible overhangs, between the *Not* I sites of pMMV.

The structure of pssDNA-Express-B is shown in Fig. 5. Mammalian cells containing the plasmid are selectable with the antibiotic zeocin. The position and general arrangement of key regions of the insert are shown in Fig. 5A and the specific arrangement of the sequences of the insert are shown in Fig. 5B. The exact positions of the structural features are shown in Fig. 5C. Transcription of the insert region is driven by the cytomegalovirus promoter and terminated in the BGH polyA region. The RNA transcript contains the MoMuLV core promoter along with some flanking regions of this promoter, the positions of which are indicated in Fig. 5B. Reverse transcriptase synthesizes a copy of the (+) (top) strand, using the tRNA pro as a primer, beginning at the position of the core promoter. Digestion of the RNA strand by RNAse H releases a single-stranded DNA sequence containing the complementary inverted repeats IR-L and IR-R (Fig. 1). Duplex formation by these repeats, shown in Fig. 2, creates a stem-loop with the sequence of interest in the loop. The stem contains a recognition site for Mbo II, GAAGA, positioned so that the enzyme, which cleaves 8/7 bases 3' to the GAAGA recognition site, releases the sequence of interest from the flanking vector sequences.

To obtain the B plasmid pTest, from which a sequence of interest that served as a control sequence is expressed, the double-stranded oligodeoxynucleotide formed by the annealed synthetic, single stranded oligodeoxynucleotides Seq. ID 11/ODN-Test(+) and Seq. ID 12/ODN-Test(-), which have compatible *Not* I ends, was inserted between the *Not* I sites of pMMV. The B plasmid expressing the telomeric repeat sequence pTelo as the sequence of interest was obtained in similar fashion, inserting the annealed, *Not* I compatible oligodeoxynucleotides Seq. ID 13/ODN-Telo(+) and Seq. ID 14/ODN-Telo(-) between the Not I sites of pMMV (Fig. 6A). The latter linker sequence contains nine repeats of the vertebrate telomere sequence 5'-AGGGTT-3' (Fig. 6B). Blackburn, E.H., 350 Nature 569-573 (1991). A third B plasmid, termed pMN-new-link, in which the stem was comprised of 29 base pairs (rather than the 27 base pair stem site of pMNV) was constructed because the 29 base pair stem gives a more stable stem-loop structure in the stem-loop intermediate than the 27 base pair stem site of pMNV. pNM-new-link was formed by ligating two self-complementary ODN's, ODN-NM-new-link(+) and ODN-NM-new-link(-), between the two *Not* I sites within pMNV.

The A plasmid, pss-DNA-Express-A (Fig. 4), was prepared from pBK-RSV (Stratagene) using XL-1 Blue MRF' as the host cell. A mouse cell line expressing Moloney murine leukemia virus was obtained from the American Type Culture Collection (#CRL-1858). The virus RNA was isolated and prepared for reverse transcriptase-PCR (RT-PCR). A 2.4 kb fragment containing the coding sequence of MoMuLV-RT was PCR-amplified using primers as set out in Seq. ID 1/ODN-RT(-) (primer position at nucleotide #2545) and Seq. ID 2/ODN-RT(+) (primer position at nucleotide #4908) to produce a DNA fragment with a 5'-Sac I and a 3'-Hind III compatible end. The 2.4 kb product obtained includes the sequence of the MoMuLV genome between positions 2546 and 4908. The mature virus reverse transcriptase peptide is encoded by the sequence between positions 2337 and 4349 (Petropoulos, C.J., Retroviral taxonomy, protein structure, sequences and genetic maps, *in* J.M Coffin (Ed.), Retroviruses, 757, Appendix 2, New York: Cold Spring Harbor Press (1997)), but peptides truncated at the amino terminus retain full activity (N. Tanese, *et al.,* 85 Proc. Natl. Acad. Sci. USA 1777-1781 (1998)). The peptide encoded by this construct includes a part of the integrase gene, which follows the reverse transcriptase in the MoMuLV polyprotein, but is not relevant here such that the length of the construct was selected because of the availability of a convenient restriction site for cloning.

The bacterium *Moraxella bovis*, which encodes the restriction endonuclease MboII (Bocklage, H., *et al.,* 19 Nucleic Acids Res. 1007-1013 (1991)), was obtained from the American Type Culture Collection (ATCC#10900). Genomic DNA was isolated from *M. bovis* and used as the template DNA in the PCR. A 1.2 kb fragment containing the *Mbo* II gene was amplified by PCR using as primers Seq. ID 3/ODN-Mbo(+) (primer position at nucleotide #887) and Seq. ID 8/ODN-Mbo(-) (primer position at nucleotide #2206). These primers contain mismatches designed to introduce a *Hind* III site into the 5' primer and an *Xba* I site into the 3' downstream primer. The 1.2 kb DNA amplification product, copying the *M. bovis* genome between positions 888 and 2206, therefore contains the coding region for the Mbo II protein. The amplification product was digested with Hind III and Xba I.

pBK-RSV was digested with Xba I and Nhe I to remove the promoter region. The *Nhe* I end was converted to a *Sac* I end using the linker formed by annealed oligodeoxynucleotides Seq. ID 6/ODN-N>S(+) and Seq. ID 7/ODN-N>S(-). The reverse transcriptase and Mbo II amplimers were ligated through the Hind III sites and this construct was subsequently ligated between the *Sac* I and *Xba* I sites of pBK-RSV to produce pBK-RSV-RT/Mbo.

To insert a flexible linker between the reverse transcriptase and Mbo II domains of the polyprotein and to provide a tag useful for purification of the protein, the double-stranded sequence formed by annealing the oligodeoxynucleotides Seq. ID 9/ODN-HisPro(+) and Seq. ID 10/ODN-HisPro(-), encoding alternate histidine and proline amino acids was inserted into pBK-RSV-RT/Mbo by digesting with Hind III. The his-pro linker, with compatible *Hind* III ends, was inserted at the *Hind* III site to produce plasmid pBK-RSV-RT/Mbo-L and the orientation was confirmed by sequencing. However, sequencing pBK-RSV-RT/Mbo-L revealed a frame shift mutation at the 5'-end of the Mbo II domain. This mutation was corrected, and the extraneous part of the integrase gene of MoMuLV was simultaneously removed, by excising the fragment of the plasmid lying between the *Ase* I and *Bgl* II sites, which encodes the 5'-end of the *Mbo* II gene, the his-pro linker region, and the integrase gene fragment, and replacing with an insert containing a modified his-pro linker and *5'-Mbo* II gene fragment. The modified his-pro linker increased the number of histidines by one, to six, and included at the 5'-end a number of unique restriction sites. The 5'-end of the Mbo II gene was modified to replace the leucine at the N-terminus that was introduced by the mismatch in the PCR primer to the original methionine and to optimize codon usage for expression of this segment of the gene in mammalian cells. The repair construct was obtained by mutually-primed DNA synthesis from two templates, ODN-Rep(+) and ODN-Rep(-), that have complementary sequences of 16 bases at the 3'-ends. These oligodeoxynucleotides were annealed and extended with the modified SEQUENASE^{™} DNA polymerase enzyme (United States Biochemical Corp.). The double-stranded product was digested with Ase I and Bgl II and inserted into the plasmid to give pssDNA-Express-A (plasmid A).

The structure of pssDNA-Express-A is shown in Fig. 8A. As set out above, to construct this plasmid, sequences encoding an active fragment of the MoMuLV reverse transcriptase and the *M. bovis* Mbo II restriction enzyme were cloned between the *Nhe* I and *Xma* I sites of the eukaryotic expression vector pBK-RSV. Transcription of the cloned region is driven by the RSV promoter and selection for transformed cells is carried out in the presence of the antibiotic G418 (neomycin). Reverse transcriptase and Mbo II are expressed as a single, bifunctional protein chain with the two functional domains separated by a short, histidine and proline rich linker.

**Tissue culture studies**. Stable and transient transfections were carried out by using lipofectant (Boehringer Mannhiem Corp.) using the manufacturer's accompanying instructions. All plasmid constructs were transfected into HeLa cell lines. Assays for ssDNA were performed by PCR and by dot-blot analyses 24-48 hours after transfection. Reverse transcriptase activity was assayed using the RT-PCR assay developed by Silver, J., *et al.* (21 Nucleic Acids Res. 3593-3594 (1993)) after transfection with pssDNA-Express-A plasmid (Fig. 11, panel A). Individual colony isolates of stably substituted HeLa cell lines (A12 and B12) were additionally assayed for RT activity (Fig. 11B). The ss-cDNA was isolated from cells transfected 48-72-hr earlier. As described above, the ss-cDNA, which co-localizes with RNA, was carried out using trizol reagent (Gibco Life Technologies, Gaithersburg, MD). Assays for specific ss-cDNA species were carried out by both PCR based assays (Fig. 12 for pTest and Fig. 13 for pTelo) for internal fragment and by denatured single stranded gel electrophoresis with subsequent nylon blotting and probing with an internal biotin-labeled probe.

This experiment showed that human tissue culture cells (HeLa and Cos-7 cell lines), co-transfected with A and B plasmids, produced ss-cDNA of the predicted size. Those skilled in the art, however, will recognize that a single plasmid including the RNA template for the stem-loop intermediate and the genes for reverse transcriptase and restriction endonuclease can also be used for this purpose as described in other examples set out herein.

The vector pNM-New-Link (which after single stranded conversion contains the more stable 29 base pair stem structure) was used for *in vitro* experiments to demonstrate premature termination of the reverse transcriptase cDNA transcript at the 3' aspect of the stem structure. As shown in Fig. 14, there was also some "read through" of this transcript through the stem structure as well (see the larger bands in Fig. 14). The sequence of interest produced from this premature termination is the second sequence of interest referred to herein.

### EXAMPLE 4. In vivo Synthesis of ss-cDNA Including DNA Enzyme in Eukaryotic Cells

The following *in vivo* experiments were designed to test whether the vector system of the present invention can be utilized to produce ssDNA including a DNA enzyme sequence in eukaryotic tissue culture cells.

**Plasmid constructs.** ODNs were prepared and plasmids constructed in the same manner as described in Example 3, above.

**Construction of B Plasmid.** A second embodiment of the first of the two plasmids comprising a two plasmid embodiment of the vector system of the present invention is the "4B" plasmid. The 4B plasmid, like plasmid pssDNA-Express-B described in Example 3, was derived from pcDNA3.1/Zeo(+) (Invitrogen Corp.), shown in Fig. 7A. pssDNA-Express-4B was constructed by digesting with restriction endonucleases Hind III and Not I at positions 911 and 978, respectively. The double-stranded linker region having compatible Hind III and Not I ends which is formed by annealing the synthetic, single stranded oligodeoxynucleotides ODN-5'-N/M(link)2-H/N and ODN-3'-N/M(link)2-H/N was ligated under standard conditions into the digested pcDNA3.1/Zeo(+) transformed into Sure II cells (Stratgene, Inc.). The ODNs were allowed to hybridize in 1 µl (5 µg/µl in water) in Ependorf tubes that were incubated at 70°C for 5 minutes and allowed to hybridize for 15 minutes at room temperature. Appropriate clones were selected and sequenced to assure proper insertion of the linker region. The resulting plasmid was termed pcDNA3.1/Zeo(+)/NM-link2-gag and re-named pssDNA-Express-4B. pssDNA-Express-4B is shown in Fig. 7B and is the plasmid into which the sequence of interest is cloned. For cloning sequences of interest between the inverted tandem repeats, the two Not I sites as positions 935 and 978, respectively (see Fig. 7B), were used. These two sites are contained within the inverted tandem repeats. For inserting sequences of interest between the inverted tandem repeats and the primer binding site, two convenient restriction endonuclease sites, Pac I and Bam H I, at positions 1004 and 1021, respectively, were used.

**Construction of A Plasmid.** The second plasmid of the two plasmid system which comprises this second embodiment of the vector of the present invention is the A plasmid, pssDNA-Express-A, shown in Fig. 8A, prepared as described in Example 3.

**Construction of C Plasmid.** As noted above, the vector system of the present invention may also take the form of a single plasmid. To produce a single plasmid vector system, or "C" plasmid, plasmid pssDNA-Express-A was digested with Sac I Xma I to remove the Mbo II gene (Fig. 8B). A linker region comprised of oligonucleotides 5'-(link)2-Hind/Xba and 3'-(link)2-Hind/Xba (Table 1), which were allowed to anneal at 70°C for 15 minutes and slowly cooled to room temperature, was ligated into the plasmid after digestion under standard conditions. Positive clones were harvested and sequenced to verify linker placement and this plasmid was then digested with Xba and Hind III. The plasmid pssDNA-Express-B was then digested with Hind III and Xba and the corresponding 300 base pair DNA fragment containing the previously described inverted tandem repeats, multiple cloning site, and PBS was cloned into the digested plasmid to give pssDNA-Express-C (Fig. 9A). Standard ligation reactions were performed and transformed into Sure II cells (Stratagene, Inc.). Transformed positive colonies were harvested and positive clones were identified by restriction analysis.

The sequences of interest were cloned into the multiple cloning site of pssDNA-Express-C by using the Bam H I and Pac I sites in the multiple cloning site (Fig. 9B). Four different sequences of interest as listed in Table 1, each including the "10-23 DNA enzyme" inserted between the 5' and 3' aspects of the antisense sequence (Fig. 9C) and shown in Figs. 10A-10D, were synthesized for these constructs, and similar procedures were utilized for inserting each of the four sequences of interest. Each construct was prepared by allowing the paired oligonucleotides to anneal at 70°C for 15 minutes and cooling to room temperature, followed by ligation into the plasmid under standard conditions. After transformation into Sure II cells, appropriate colonies were selected with verification by sequencing for the individual inserts. The antisense ability of each of these plasmids was tested by transfecting each plasmid, with appropriate controls which contained a random sequence of equal length and did not contain antisense inserts or the "10-23 DNA enzyme," into HeLa cells using lipofectant reagent (Boehringer Mannheim) under standard conditions. Trizol reagent was used to harvest the cells and RNA fraction, and subsequent Northern blot analysis was performed to demonstrate specific antisense expression.

**Tissue culture studies.** Stable and transient transfections were carried out by using lipofectant (Boehringer Mannhiem Corp.) using the manufacturer's accompanying instructions. All plasmid constructs were transfected into HeLa cell lines. Assays for ssDNA were performed by PCR and by dot-blot analyses 24-48 hours after transfection. Reverse transcriptase activity was assayed using the RT-PCR assay developed by Silver, J., *et al., supra,* after transfection with pssDNA-Express-A plasmid. Individual colony isolates of stably substituted HeLa cell lines (A12 and B12) were additionally assayed for RT activity. The ss-cDNA was isolated from cells transfected 48-72-hr earlier using trizol reagent. Assays for specific ss-cDNA species were carried out by both PCR based assays for internal fragment and by denatured single stranded gel electrophoresis with subsequent nylon blotting and probing with an internal biotin-labeled probe.

This experiment showed that human tissue culture cells (HeLa cell line), transfected with plasmids designed to synthesize a processed ss-cDNA, produced ss-cDNA of the predicted size. As described in Example 3, the ssDNA sequence of interest produced in accordance with the method of the present invention is produced from either the position between the inverted repeats after digestion of the stem of the stem-loop intermediate or from the position between the inverted repeats and the primer binding site by premature termination of the reverse transcriptase cDNA transcript at the 3' aspect of the stem structure. The sequence of interest produced from this premature termination is the second sequence of interest referred to herein. Fig. 15 shows that cells transfected with plasmid pssDNA-Express-4B having the 10-23 enzyme included in the antisense sequence against c-raf kinase which was utilized as the sequence of interest produced an antisense sequence against c-raf kinase that included the 10-23 DNA enzyme from the position between the inverted tandem repeats and the primer binding site.

The experiments described above demonstrate a method of production of ssDNA *in vitro* and *in vivo* by multiple stepwise reactions using eukaryotic reverse transcriptase reactions and various cDNA priming reactions. This reaction was followed by formation of a "stem-loop" intermediate which can be used to eliminate any unwanted sequences either upstream 5' or downstream 3' from a designed (and formed) "stem" after being subsequently cleaved by a restriction endonuclease.

Any nucleotide sequence of interest could be produced by this method in a eukaryotic cell. This sequence of interest is cloned (or synthesized) between the designed inverted tandem repeats and represents the sequence in the "loop" after ssDNA production and subsequent stem-loop formation. The sequence of interest to be produced can be of any base (i.e., A,T,G,C) composition as long as the sequence does not interfere with the formation of the stem of the stable stem-loop intermediate, which may optionally include functional genetic element such as a specific enzyme recognition sequence, for instance, a site which serves as a substrate for a particular restriction endonuclease. Again, any restriction endonuclease may also be used to digest (or cleave) the stem portion of the stem-loop intermediate as long as the recognition site for that particular restriction endonuclease has been designed into the inverted tandem repeat.

Although described with reference to the figures and specific examples set out herein, those skilled in the art will recognize that certain changes can be made to the specific elements set out herein without changing the manner in which those elements function to achieve their intended respective results. For instance, the cassette described herein is comprised of three genetic elements, a sequence of interest, a tandem inverted repeat, and a primer binding site. Other genetic elements include an optional restriction endonuclease gene and a reverse transcriptase gene, each of these genes being provided with appropriate promoters as described herein. Those skilled in the art will recognize that, for instance, the mouse Moloney leukemia virus reverse transcriptase gene described for use as the reverse transcriptase gene of the cassette can be replaced with other reverse transcriptase genes (the reverse transcriptase gene from Human immunodeficiency virus was one such gene which was noted above) and that promoters other than the CMV promoter may be used to advantage. Further, several restriction endonuclease genes are listed above, but those skilled in the art will recognize from this description that the list set out above is not exhaustive and that many other restriction endonuclease genes will function to advantage in connection with the present invention. Similarly, the RSV promoter described as being used in connection with the restriction endonuclease genes set out herein is not the only promoter which may be used to advantage. All such changes and modifications which do not depart from the spirit of the present invention are intended to fall within the scope of the following non-limiting claims.

**Table #1**

| **Oligodeoxynucleotides (ODN's)** |
|---|
| Name: 5'-N/M(link)2-H/N |
| |
| Name: 3'- N/M(link)2-H/N |
| |
| Name: 5'- polyNM-gaglink-(Pleio)-DNAse-1023-B/P |
| 5'-GAT GTA AG TCG TTG TAG CTA GCC TCC CCT G -3' |
| Name: 3'-polyNM-gaglink-(Pleio)-DNAse-1023-B/P |
| 5'-GAT CCA GGG GA GGC TAG CTA CAA CGA CTT ACA TCA T -3' |
| Name: 5'-polyNM-gaglink-(hras)-DNAse-1023-B/P |
| 5'-GGTGGG CGCCTCGTTGTAGCTAGCCTCGGTGTGGG-3' |
| Name: 3'- polyNM-gaglink-(hras)-DNAse-1023-B/P |
| 5'-GATCCCCACACCGAGGCTAGCTACAACGAGGCGCCCACCAT-3' |
| Name: 5'-polyNM-gaglink-(rafK)-DNAse-1023-B/P |
| 5'-AATGCATGTCTCGTTGTAGCTAGCCCAGGCGGGA-3 |
| Name: 3'- polyNM-gaglink-(rafK)-DNAse-1023-B/P |
| 5'-GATCTCCCGCCTGGGCTAGCTACAACGAGACATGCATTAT-3' |
| Name: 5'-polyN/M-gaglink-(tat-SIV)-DNAse-1023-B/P |
| 5'-AGATGGAGACTCGTTGTAGCTAGCCCCCTTGAGGGCAGATTGGCGCCCGAACAGGGACTTGAAGGA-3' |
| Name: 3'- polyN/M-gaglink-(tat-SIV)-DNAse-1023-B/P |
| 5'-GATCTCCTTCAAGTCCCTGTTCGGGCGCCAATCTGCCCTCAAGGGGGCTAGCTACAACGAGTCTCCATCTAT-3' |
| Name: 5'-(LINK)2-Hind/Xba |
| 5'-CCG GAT CTA GAC CGC AAG CTT CAC CGC -3' |
| Name: 3'-(LINK)2-Hind/Xba |
| 5'-GGT GAA GCT TGC GGT CTA GAT -3' |

| | |
|---|---|
| ODN-PMMV(+) 129 bases (#23) | |
| ODN-PMMV(-) 121 bases (#24) | |
| ODN-Test (+) 57 bases (#38) | |
| ODN-Test (-) 57 bases (#39) | |
| ODN-Telo (+) 92 bases (#40) | |
| ODN-Telo (-) 92 bases (#41) | |
| ODN-XB(+) 51 bases | |
| ODN-XB(-) 51 bases | |
| ODN-RT (+). 32 bases (#13) | 5'-GGGATCAGGAGCTCAGATCATGGGACCAATGG-3' |
| ODN-RT (-) 24 bases (#12) | 5'-CTTGTGCACAAGCTTTGCAGGTCT-3' |
| ODN-N>S (+) 18 bases (#25) | 5'-CTAGCGGCAAGCGTAGCT-3' |
| ODN-N>S (-) 10 bases (#26) | 5'-ACGCTTGCCG-3' |
| ODN-Mbo (+) 30 bases (#16) | 5'-CAATTAAGGAAAGCTTTGAAAAATTATGTC-3' |
| ODN-Mbo (-) 27 bases (#33) | 5'-TAATGGCCCGGGCATAGTCGGGTAGGG-3' |
| ODN-HisPro (+) 43 bases (#36) | 5'-AGCTGGATCCCCCGCTCCCCACCACCACCACCACCCTGCCCCT-3' |
| ODN-HisPro (-) 42 bases (#37) | 5'-AGCAGGGGCAGGGTGGTGGTGGTGGTGGGGAGCGGGGGATCC-3' |
| ODN-Rep(+) 121 bases | |
| ODN-Rep(-) 111 bases | |

1. Name: 3'-RT/Mol-Hind III (24-mer)
   Sequence: 5'-CTT GTG CAC AAG CTT TGC AGG TCT-3'
2. Name: 5'-RT/Mol-Sac I (32-mer)
   Sequence: 5'-GGG ATC AGG AGC TCA GAT CAT GGG ACC AAT GG-3'
3. Name: 5'-Mbo II-Hind III (30-mer)
   Sequence: 5'-CAA TTA AGG AAA GCT TTG AAA AAT TAT GTC-3'
4. Name:5'-RT-Not-Mbo-Link (129-mer)
5. Name: 3'-RT-Not-Mbo-Link (121-mer)
6. Name: 5'-Nhe-Sac-Link (18-mer)
   Sequence: 5'-CTA GCG GCA AGC GTA GCT-3'
7. Name: 3'-Nhe-Sac-Link (10-mer)
   Sequence: 5'-ACG CTT GCC G-3'
8. Name: 3'-Mbo II-Xba I (27-mer)
   Sequence: 5'-TAA TGG CCC GGG CAT AGT CGG GTA GGG -3'
9. Name: 5'-Hind-link-Histag(43-mer)
   Sequence: 5'-A GCT GGA TCC CCC GCT CCC CAC CAC CAC CAC CAC CCT GCC CCT-3'
10. Name: 3'-Hind-link-Histag (42-mer)
   Sequence: 5'-AGC AGG GGC AGG GTG GTG GTG GTG GTG GGG AGC GGG GGA TCC-3'
11. Name: 5'-Not-link-test1 (57-mer)
12.. Name:3'-Not-link-testl (57-mer)
13. Name: 5'-Not-Mbo-link-telo (92-mer)
14. Name: 3'-Not-Mbo-link-telo (92-mer)
15. 5'-SL-linker-Fok1-RT (111-mer)
16. 3'-SL-linker-Fok1-RT (103-mer)

## Claims

1. A nucleic acid construct for delivery into a cell which comprises:
3' and 5' complementary sequences comprising an inverted tandem repeat,
a sequence encoding a primer binding site for a reverse transcriptase that is complementary to a transfer RNA (tRNA) and located in a 3' position with respect to the inverted tandem repeat, and
a sequence coding for a sequence of interest located either between the 3' and 5' complementary sequences of the inverted tandem repeat, or between the inverted tandem repeat and the sequence encoding the 3' primer binding site.

2. A nucleic acid construct according to Claim 1, wherein the sequence encoding the sequence of interest is located between the 3' and 5' complementary sequences of the inverted tandem repeat.

3. A nucleic acid construct according to Claim 2 which comprises a second sequence coding for a sequence of interest located between the inverted tandem repeat and the sequence encoding the 3' primer binding site.

4. A nucleic acid construct according to Claim 1, wherein the sequence encoding the sequence of interest is located between the inverted tandem repeat and the sequence encoding the 3' primer binding site.

5. A nucleic acid construct according to any one of Claims 2 to 4, wherein the inverted tandem repeat is capable of forming a stem-loop intermediate in a single stranded nucleic acid product encoded by said nucleic acid construct, with said-inverted tandem repeat forming the stem of said stem-loop intermediate.

6. A nucleic acid construct according to any one of Claims 2 to 5, wherein the inverted tandem repeat comprises a sequence encoding one or more specific enzyme recognition sequence(s).

7. A nucleic acid construct according to Claim 6, wherein the specific enzyme recognition sequence comprises a restriction endonuclease site.

8. A nucleic acid construct according to Claim 7, further comprising a gene encoding a restriction endonuclease.

9. A nucleic acid construct according to Claim 8, wherein the restriction endonuclease gene is located in a 5' position with respect to the inverted tandem repeat.

10. A nucleic acid construct according to any one of Claims 6 to 9, wherein the specific enzyme recognition sequence comprises a Hind III or a Not I restriction site, or a recognition sequence for a restriction endonuclease selected from the group consisting of endonuclease type I, endonuclease type II, and endonuclease type III.

11. A nucleic acid construct according to any one of Claims 1 to 10, wherein the inverted tandem repeat comprises a sequence encoding one or more eukaryotic, prokaryotic, and/or viral protein DNA binding sites.

12. A nucleic acid construct according to anyone of Claims 1 to 11, wherein the inverted tandem repeat acts in *cis*-oriented fashion.

13. A nucleic acid construct according to any one of Claims 1 to 12, wherein the primer binding site is specific for an endogenous reverse transcriptase.

14. A nucleic acid construct according to any one of Claims 1 to 12, further comprising a gene encoding a reverse transcriptase or a reverse transcriptase/RNAse H polyprotein.

15. A nucleic acid construct according to Claim 14, wherein the gene encoding the reverse transcriptase or reverse transcriptase/RNAse H polyprotein is located in a 5' position with respect to the inverted tandem repeat.

16. A nucleic acid construct according to Claim 14 or 15, wherein the gene encoding the reverse transcriptase/RNAse H polyprotein is from Moloney murine leukemia virus, human immunodeficiency virus, or simian immunodeficiency virus.

17. A nucleic acid construct according to any one of Claims 14 to 16, wherein the primer binding site is specific for a reverse transcriptase encoded by the reverse transcriptase or reverse transcriptase/RNAse H polyprotein gene.

18. A nucleic acid construct according to any one of the preceding claims, further comprising a promoter for each of said first or second sequences encoding a sequence of interest, said restriction endonuclease, said reverse transcriptase or said reverse transcriptase/RNase H gene.

19. A nucleic acid construct according to Claim 18, wherein the promoter and/or enhancer is a eukaryotic promoter.

20. A nucleic acid construct according to Claim 18 or 19, wherein the promoter is a constitutive, inducible, wide-spectrum or tissue specific promoter.

21. A nucleic acid construct according to any one of the preceding claims, further comprising a sequence which encodes a polyadenylation tail sequence located in a 3' position with respect to the 3' primer binding site.

22. A nucleic acid construct according to any one of the preceding claims, wherein the first or second sequence encoding a sequence of interest includes a sequence which encodes a ssDNA having enzymatic activity.

23. A nucleic acid construct according to Claim 22, wherein the first or second sequence encoding a sequence of interest includes the sequence 5'-GGCTAGCTACAACGA-3', flanked in both the 5' and 3' directions by sequences encoding one or more sequence(s) complementary to a target mRNA species.

24. A nucleic acid construct according to Claim 23, wherein the target mRNA species is for:
(i) h-ras,
(ii) c-raf kinase,
(iii) pleiotrophin angiogenic growth factor, or
(iv) the tat region of simian immunodeficiency virus (SIV).

25. A set of genetic elements adapted for incorporation into a vector for delivery to a cell comprising:
(i) a cassette comprising a nucleic acid construct according to any one of Claims 2 to 4; and
(ii) a gene encoding a reverse transcriptase.

26. The set of genetic elements of Claim 25 wherein said reverse transcriptase gene is selected from the group consisting of the reverse transcriptase genes from Moloney murine leukemia virus or human immunodeficiency virus.

27. A set of genetic elements according to Claim 25 comprising (a) a sequence coding for a sequence of interest flanked by 3' and 5' complementary sequences comprising an inverted tandem repeat, (b) a sequence encoding a primer binding site (PBS) for a reverse transcriptase in a 3' position with respect to the inverted tandem repeat, whereby the PBS consists of a nucleic acid sequence complementary to a transfer RNA (tRNA) sequence which is resident within a eucaryotic target cell, and (c) a gene encoding a reverse transcriptase/RNAse H and restriction endonuclease.

28. A nucleic acid construct according to any one of Claims 1 to 24, or a set of genetic elements according to any one of Claims 25 to 27, wherein the sequence of interest is a single stranded nucleic acid molecule.

29. A nucleic acid construct or set of genetic elements according to Claim 28, wherein the single stranded nucleic acid molecule is cDNA or an mRNA.

30. A nucleic acid construct or set of genetic elements according to any one of Claims 28 or 29, wherein the single stranded nucleic acid molecule is an inhibitory nucleic acid molecule.

31. A nucleic acid construct or set of genetic elements according to Claim 30, wherein the inhibitory nucleic acid molecule is an antisense sequence or aptamer.

32. A nucleic acid construct or set of genetic elements according to any one of Claims 1 to 31, wherein the nucleic acid is DNA.

33. An mRNA transcript comprising (a) 3' and 5' complementary sequences comprising an inverted tandem repeat, (b) a primer binding site that is complementary to a transfer RNA (tRNA) and located 3' to the inverted tandem repeat, and (c) a sequence coding for a sequence of interest located either between the 3' and 5' complementary sequences of the inverted tandem repeat, or between the inverted tandem repeat and the 3' primer binding site.

34. An mRNA transcript according to Claim 33 wherein the sequence coding for the sequence of interest is located between the inverted tandem repeat and the 3' primer binding site.

35. The mRNA transcript of any one of Claims 33 or 34, wherein the sequence encoding the sequence of interest includes a sequence encoding a ssDNA having enzymatic activity.

36. An mRNA of Claim 33, which is a transcript of the nucleic acid construct of Claim 32.

37. A ssDNA transcript of the mRNA of any one of Claims 33 to 36.

38. A vector which comprises:
3' and 5' complementary sequences comprising an inverted tandem repeat,
a sequence encoding a primer binding site for a reverse transcriptase that is complementary to a transfer RNA (tRNA) and located in a 3' position with respect to the inverted tandem repeat, and
an insertion site for a sequence coding for a sequence of interest between the 3' and 5' complementary sequences of the inverted tandem repeat, or between the inverted tandem repeat and the sequence encoding the 3' primer binding site.

39. A vector according to Claim 38, which comprises a first insertion site between the 3' and 5' complementary sequences of the inverted tandem repeat and a second insertion site between the inverted tandem repeat and the sequence encoding the 3' primer binding site.

40. A vector according to Claim 38 or 39, further comprising a gene encoding a reverse transcriptase or a reverse transcriptase/RNAse H polyprotein.

41. A vector according to Claim 40, wherein the reverse transcriptase or reverse transcriptase/RNAse H polyprotein gene is located in a 5' position with respect to the inverted tandem repeat.

42. A vector according to Claim 38 comprising the nucleic acid construct or set of genetic elements of any one of Claims 1 to 32.

43. A vector system which comprises a first vector according to Claim 38 or 39, and a second vector comprising a gene which encodes a reverse transcriptase.

44. A vector or vector system according to any one of Claims 38 to 43, wherein the vector is a plasmid or modified viral construct.

45. A vector or vector system according to any one of Claims 38 to 44, wherein the gene is operably linked to an expression control sequence.

46. A host cell stably transformed or transfected with a vector or vector system according to any one of Claims 38 to 45 or comprising a transcript of any one of Claims 33 to 37.

47. A host cell according to Claim 46 which is a eukaryotic or bacterial cell.

48. An *in vitro* method of producing a single-stranded nucleic acid molecule having a sequence of interest, which method comprises the steps of introducing a nucleic acid construct or set of genetic elements according to any one of Claims 1 to 32 into a target cell, transcribing the nucleic add construct or set of genetic elements into mRNA, and reverse transcribing the mRNA transcript into cDNA.

49. A method according to Claim 48, wherein reverse transcription is carried out by a reverse transcriptase which is endogenous to the target cell.

50. A method according to Claim 48, further comprising the step of introducing a gene encoding a reverse transcriptase or a reverse transcriptase/RNAse H polyprotein into the target cell.

51. A method according to any one of Claims 48 to 50, further comprising the step of linearising the cDNA transcript by cutting the cDNA stem-loop structure formed by the inverted tandem repeat where the loop structure joins the stem.

52. The method of Claim 51 wherein the cDNA is linearized by including a restriction endonuclease site in the inverted tandem repeat, the cDNA forming a stem-loop intermediate by Watson-Crick base pairing of the inverted tandem repeat, and cutting the stem of the stem-loop intermediate with a restriction endonuclease.

53. A method according to Claim 52, further comprising the step of introducing a gene encoding a restriction endonuclease or a gene encoding a reverse transcriptase/RNAse H polyprotein linked via a proline rich linker to a restriction endonuclease into the target cell.

54. The method of any one of Claims 48 to 53 additionally comprising inducibly promoting the transcription of the reverse transcriptase gene.

55. A method according to Claim 48, comprising the steps of introducing the set of genetic elements of Claim 27 into a target cell, transcribing the set of genetic elements into mRNA, reverse transcribing the mRNA transcript of said cassette with the reverse transcriptase in the nucleus of human tissue culture cells, digesting the resulting heteroduplex with the RNAse H and removing flanking sequences by either (a) restriction endonuclease digestion of the stem loop intermediate or (b) by premature termination of the cDNA transcript by formation of a stem-loop secondary structure by the self complementary inverted tandem repeats.

56. A pharmaceutical composition which comprises a nucleic acid construct or set of genetic elements according to any one of Claims 1 to 32, a vector or vector system according to any one of Claims 38 to 45, or a host cell according to Claim 46 or 47, together with a pharmacologically acceptable adjuvant, diluent or carrier.

57. A nucleic acid construct or set of genetic elements according to any one of Claims 1 to 32, a vector or vector system according to any one of Claims 38 to 45, or a host cell according to Claim 46 or 47, for use in therapy, especially for use in delivering an inhibitor nucleic acid molecule to a target cell.

58. A nucleic acid construct or set of genetic elements according to Claim 57, wherein the nucleic acid construct or set of genetic elements is for use in an *in vivo* method of producing a single-stranded nucleic acid molecule having a sequence of interest, which method comprises the steps of introducing the nucleic acid construct or set of genetic elements into a target cell, transcribing the nucleic acid construct into mRNA, and reverse transcribing the mRNA transcript into cDNA.

59. A nucleic acid construct or set of genetic elements according to Claim 58, wherein reverse transcription is carried out by a reverse transcriptase which is endogenous to the target cell.

60. A nucleic acid construct or set of genetic elements according to Claim 58, wherein the method further comprises the step of introducing a gene encoding a reverse transcriptase or a reverse transcriptase/RNAse H polyprotein into the target cell.

61. A nucleic acid construct or set of genetic elements according to any one of Claims 58 to 60, wherein the method further comprises the step of linearising the cDNA transcript by cutting the cDNA stem-loop structure formed by the inverted tandem repeat where the loop structure joins the stem.

62. A nucleic acid construct or set of genetic elements according to Claim 61, wherein the cDNA is linearized by including a restriction endonuclease site in the inverted tandem repeat, the cDNA forming a stem-loop intermediate by Watson-Crick base pairing of the inverted tandem repeat, and cutting the stem of the stem-loop intermediate with the restriction endonuclease.

63. A nucleic acid construct or set of genetic elements according to Claim 62, which method further comprises the step of introducing a gene encoding a restriction endonuclease or a gene encoding a reverse transcriptase/RNAse H polyprotein linked via a proline rich linker to a restriction endonuclease into the target cell.

64. A nucleic acid construct or set of genetic elements according to any one of Claims 58 to 63, wherein the method additionally comprises inducibly promoting the transcription of the reverse transcriptase gene.

65. Use of a nucleic acid construct or set of genetic elements according to any one of Claims 1 to 32, a vector or vector system according to any one of Claims 38 to 45, or a host cell according to Claim 46 or 47, for the manufacture of a medicament for alleviating pathological condition by regulating gene expression, especially for alleviating a pathological condition by delivery of an inhibitory nucleic acid molecule to a target cell.

## Patentansprüche

1. Nukleinsäurekonstrukt zur Abgabe in eine Zelle, das umfasst:
3'und 5'komplementäre Sequenzen, die einen invertierten Tandem-Repeat umfassen,
eine Sequenz, die eine Primerbindungsstelle für eine Reverse Transkriptase codiert, die komplementär ist zu einer transfer RNA (tRNA) und im Hinblick auf den invertierten Tandem-Repeat in einer 3'Position lokalisiert ist, und
eine Sequenz, die für eine Sequenz von Interesse kodiert und entweder zwischen den 3'und 5'komplementären Sequenzen des invertierten Tandem-Repeat lokalisiert ist oder zwischen dem invertierten Tandem-Repeat und der Sequenz, die die 3'Primerbindungsstelle kodiert.

2. Nukleinsäurekonstrukt nach Anspruch 1, wobei die Sequenz, die die Sequenz von Interesse kodiert, zwischen den 3'und 5'komplementären Sequenzen des invertierten Tandem-Repeat lokalisiert ist.

3. Nukleinsäurekonstrukt nach Anspruch 2, das eine zweite Sequenz umfasst, die für eine Sequenz von Interesse kodiert, die zwischen dem invertierten Tandem-Repeat und der Sequenz, die die 3'Primerbindungsstelle kodiert, lokalisiert ist.

4. Nukleinsäurekonstrukt nach Anspruch 1, wobei die Sequenz, die die Sequenz von Interesse kodiert, zwischen dem invertierten Tandem-Repeat und der Sequenz, die die 3'Primerbindungsstelle kodiert, lokalisiert ist.

5. Nukleinsäurekonstrukt nach einem der Ansprüche 2 bis 4, wobei der invertierte Tandem-Repeat geeignet ist, ein Stamm-Schlaufe-Intermediat in einem einzelsträngigen Nukleinsäureprodukt zu formen, das von dem besagten Nukleinsäurekonstrukt kodiert wird, wobei der besagte invertierte Tandem-Repeat den Stamm des Stamm-Schlaufe Intermediats formt.

6. Nukleinsäurekonstrukt nach einem der Ansprüche 2 bis 5, wobei der invertierte Tandem-Repeat eine Sequenz umfasst, die für ein oder mehrere spezifische Enzym-Erkennungsstellen kodiert.

7. Nukleinsäurekonstrukt nach Anspruch 6, wobei die spezifische Enzymerkennungsstelle eine Restriktionsendonuklease-Stelle umfasst.

8. Nukleinsäurekonstrukt nach Anspruch 7, das weiterhin ein Gen umfasst, das eine Restriktionsendonuklease kodiert.

9. Nukleinsäurekonstrukt nach Anspruch 8, wobei das Restriktionsendonuklease-Gen im Hinblick auf den invertierten Tandem-Repeat in einer 5'Position angeordnet ist.

10. Nukleinsäurekonstrukt nach einem der Ansprüche 6 bis 9, wobei die spezifische Enzym-Erkennungsstelle eine Hind III oder eine Not I Restriktionsstelle umfasst, oder eine Erkennungssequenz für eine Restriktionsendonuklease, die aus der Gruppe ausgewählt ist, die aus Endonuklease Typ I, Endonuklease Typ II und Endonuklease Typ III besteht.

11. Nukleinsäurekonstrukt nach einem der Ansprüche 1 bis 10, wobei der invertierte Tandem-Repeat eine Sequenz umfasst, die eine oder mehrere eukaryotische, prokaryotische und/oder virale Protein DNA-Bindungsstellen kodiert.

12. Nukleinsäurekonstrukt nach einem der Ansprüche 1 bis 11, wobei der invertierte Tandem-Repeat in einer cis-orientierten Art und Weise funktioniert.

13. Nukleinsäurekonstrukt nach einem der Ansprüche 1 bis 12, wobei die Primerbindungsstelle spezifisch ist für eine endogene Reverse Transkriptase.

14. Nukleinsäurekonstrukt nach einem der Ansprüche 1 bis 12, das weiterhin ein Gen umfasst, das eine Reverse Transkriptase oder ein Reverse Transkriptase/RNAse H Polyprotein kodiert.

15. Nukleinsäurekonstrukt nach Anspruch 14, wobei das Gen, das die Reverse Transkriptase oder das Reverse Transkriptase/RNAse H Polyprotein kodiert, im Hinblick auf den invertierten Tandem-Repeat in einer 5'Position angeordnet ist.

16. Nukleinsäurekonstrukt nach Anspruch 14 oder 15, wobei das Gen, das das Reverse Transkriptase/RNAse H Polyprotein kodiert von dem Moloney Murine Leukemia Virus, dem humanen Immunschwäche-Virus (HIV) oder dem Simian Immunodefizienz Virus (SIV) ist.

17. Nukleinsäurekonstrukt nach einem der Ansprüche 14 bis 16, wobei die Primerbindungsstelle spezifisch ist für eine Reverse Transkriptase, die von dem Reverse Transkriptase-Gen oder dem Reverse Transkriptae/RNAse H Polyprotein-Gen kodiert wird.

18. Nukleinsäurekonstrukt nach einem der vorhergehenden Ansprüche, das weiterhin einen Promotor für jede besagte erste oder zweite Sequenz umfasst, die eine Sequenz von Interesse, das besagte Restriktions Endonuklease-Gen, das besagte Reverse Transkriptase-Gen oder das besagte Reverse Transkriptase/RNAse H Gen kodiert.

19. Nukleinsäurekonstrukt nach Anspruch 18, wobei der Promotor und/oder Enhancer ein eukaryotischer Promotor ist.

20. Nukleinsäurekonstrukt nach Anspruch 18 oder 19, wobei der Promotor ein konstitutiver, ein induzierbarer, ein Promotor mit breitem Spektrum oder ein gewebespezifischer Promotor ist.

21. Nukleinsäurekonstrukt nach einem der vorhergehenden Ansprüche, das ferner eine Sequenz umfasst, die eine Polyadenylierungs-Endsequenz kodiert, die im Hinblick auf die 3'Primerbindungsstelle in 3'Position angeordnet ist.

22. Nukleinsäurekonstrukt nach einem der vorhergehenden Ansprüche, wobei die erste oder zweite Sequenz, die eine Sequenz von Interesse kodiert, eine Sequenz einschließt, die eine einzelsträngige DNA (ssDNA) kodiert, die enzymatische Aktivität hat.

23. Nukleinsäurekonstrukt nach Anspruch 22, wobei die erste oder zweite Sequenz, die eine Sequenz von Interesse kodiert, eine Sequenz 5'-GGCTAGCTACAACGA-3' einschließt und in den beiden 5'und 3'Richtungen von Sequenzen flankiert wird, die eine oder mehrere Sequenz(en) kodieren, die komplementär sind zu einer Ziel mRNA Spezies.

24. Nukleinsäurekonstrukt nach Anspruch 23, wobei die Ziel mRNA- Spezies ist für:
(i) h-ras,
(ii) c-raf Kinase,
(iii) Pleiotrophin angiogenischen Wachstumsfaktor, oder
(iv) die tat Region des SIV.

25. Set von genetischen Elementen, die angepasst sind für das Einfügen in einen Vektor zum Einbringen in eine Zelle, umfassend:
(i) eine Kassette, die ein Nukleinsäurekonstrukt nach einem der Ansprüche 2 bis 4 umfasst; und
(ii) ein Gen, das eine Reverse Transkriptase kodiert.

26. Set von genetischen Elementen nach Anspruch 25, wobei das besagte Reverse Transkriptase-Gen ausgewählt ist aus der Gruppe, die aus den Reverse Transkriptase-Genen des Moloney Murine Leukemia Virus oder des humanen Immundefizienz-Virus besteht.

27. Set von genetischen Elementen nach Anspruch 25 umfassend (a) eine Sequenz, die für eine Sequenz von Interesse kodiert und von 3'und 5'komplementären Sequenzen flankiert wird, die einen invertierten Tandem-Repeat umfassen, (b) eine Sequenz, die eine Primerbindungsstelle (PBS) für eine Reverse Transkriptase kodiert, die hinsichtlich des invertierten Tandem-Repeat in einer 3'Position ist, wobei die PBS aus einer Nukleinsäure besteht, die komplementär ist zu einer transfer RNA (tRNA)-Sequenz, die innerhalb einer eukaryotischen Zielzelle ansässig ist, und (c) ein Gen, das eine Reverse Transkriptase/RNAse H und eine Restriktionsendonuklease kodiert.

28. Nukleinsäurekonstrukt nach einem der Ansprüche 1 bis 24, oder ein Set von genetischen Elementen nach einem der Ansprüche 25 bis 27, wobei die Sequenz von Interesse ein einzelsträngiges Nukleinsäuremolekül ist.

29. Nukleinsäurekonstrukt oder Set von genetischen Elementen nach Anspruch 28, wobei das einzelsträngige Nukleinsäuremolekül eine cDNA oder eine mRNA ist.

30. Nukleinsäurekonstrukt oder Set von genetischen Elementen nach einem der Ansprüche 28 oder 29, wobei das einzelsträngige Nukleinsäuremolekül ein inhibitorisches Nukleinsäuremolekül ist.

31. Nukleinsäurekonstrukt oder Set von genetischen Elementen nach Anspruch 30, wobei das inhibitorische Nukleinsäuremolekül eine Antisense-Sequenz oder ein Aptamer ist.

32. Nukleinsäurekonstrukt oder Set von genetischen Elementen nach einem der Ansprüche 1 bis 31, wobei die Nukleinsäure eine DNA ist.

33. mRNA-Transkript, das umfasst: (a) 3'und 5'komplementäre Sequenzen umfassend einen invertierten Tandem-Repeat, (b) eine Primerbindungsstelle, die komplementär ist zu einer transfer-RNA (tRNA) und 3'zu dem invertierten Tandem-Repeat angeordnet ist, und (c) eine Sequenz kodierend für eine Sequenz von Interesse, die entweder zwischen den 3'und 5'komplementären Sequenzen des invertierten Tandem-Repeat oder zwischen dem invertierten Tandem-Repeat und der 3'Primerbindungsstelle angeordnet ist.

34. mRNA Transkript nach Anspruch 33, wobei die Sequenz, die die Sequenz von Interesse kodiert, zwischen dem invertierten Tandem-Repeat und der 3'Primerbindungsstelle angeordnet ist.

35. mRNA Transkript nach einem der Ansprüche 33 oder 34, wobei die Sequenz, die die Sequenz von Interesse kodiert, eine Sequenz einschließt, die eine ssDNA mit enzymatischer Aktivität kodiert.

36. mRNA nach Anspruch 33, die ein Transkript des Nukleinsäurekonstrukt nach Anspruch 32 ist.

37. ssDNA Transkript von der mRNA nach einem der Ansprüche 33 bis 36.

38. Vektor, der umfasst:
3'und 5'komplementäre Sequenzen umfassend einen invertierten Tandem Repeat,
eine Sequenz, die eine Primerbindungsstelle für eine Reverse Transkriptase kodiert, die komplementär ist zu einer transfer-RNA (tRNA) und im Hinblick auf den invertierten Tandem-Repeats in einer 3'Position lokalisiert ist, und
eine Insertionsstelle für eine Sequenz, die für eine Sequenz von Interesse kodiert und zwischen den 3'und 5'komplementären Sequenzen des invertierten Tandem-Repeat lokalisiert ist, oder zwischen dem invertierten Tandem-Repeat und der Sequenz, die die 3'Primerbindungsstelle kodiert.

39. Vektor nach Anspruch 38, der eine erste Insertionsstelle zwischen den 3'und 5'komplementären Sequenzen des invertierten Tandem-Repeat umfasst und eine zweite Insertionsstelle zwischen dem invertierten Tandem-Repeat und der Sequenz, die die 3'Primerbindungsstelle kodiert.

40. Vektor nach Anspruch 38 oder 39, weiterhin umfassend ein Gen, das eine Reverse Transkriptase oder ein Reverse Transkriptase/RNAse H Polyprotein kodiert.

41. Vektor nach Anspruch 40, wobei das Gen für Reverse Transkriptase oder Reverse Transkriptase/RNAse H Polyprotein hinsichtlich des invertierten Tandem-Repeat in einer 5'Position angeordnet ist.

42. Vektor nach Anspruch 38 umfassend ein Nukleinsäurekonstrukt oder ein Set von genetischen Elementen nach einem der Ansprüche 1 bis 32.

43. Vektorsystem, das einen ersten Vektor nach Anspruch 38 oder 39 umfasst und einen zweiten Vektor umfassend ein Gen für eine Reverse Transkriptase.

44. Vektor oder Vektorsystem nach einem der Ansprüche 38 bis 43, wobei der Vektor ein Plasmid oder ein modifiziertes virales Konstrukt ist.

45. Vektor oder Vektorsystem nach einem der Ansprüche 38 bis 43, wobei das Gen nutzbar an eine Expressionskontrollsequenz gebunden ist.

46. Wirtszelle stabil transformiert oder transfiziert mit einem Vektor oder einem Vektorsystem nach einem der Ansprüche 38 bis 45 oder umfassend ein Transkript nach einem der Ansprüche 33 bis 37.

47. Wirtszelle nach Anspruch 46, die eine eukaryotische Zelle oder eine Bakterienzelle ist.

48. *In vitro* Verfahren zur Herstellung eines einzelsträngigen Nukleinsäuremoleküls, das eine Sequenz von Interesse hat, wobei das Verfahren die Schritte des Einführens eines Nukleinsäurekonstrukts oder eines Set von genetischen Elementen nach einem der Ansprüche 1 bis 32 in eine Zielzelle,
der Transkription des Nukleinsäurekonstrukts oder des Sets von genetischen Elementen in mRNA, und
der reversen Transkription des mRNA Transkripts in cDNA umfasst.

49. Verfahren nach Anspruch 48, wobei die Reverse Transkription durch eine Reverse Transkriptase ausgeführt wird, die endogen zu der Zielzelle ist.

50. Verfahren nach Anspruch 48, weiterhin umfassend den Schritt des Einführens eines Gens in eine Zielzelle, das eine Reverse Transkriptase oder ein Reverse Transkriptase/RNAse H Polyprotein kodiert.

51. Verfahren nach einem der Ansprüche 48 bis 50, weiterhin umfassend den Schritt der Linearisierung des cDNA Transkripts durch Schneiden der cDNA Stamm-Schlaufe Struktur, die von dem invertierten Tandem-Repeat geformt wird, in dem die Schlaufenstruktur den Stamm verbindet.

52. Verfahren nach Anspruch 51, wobei die cDNA, die ein Stamm- Schlaufe Intermediat durch Watson-Crick Basenpaarung des invertierten Tandem-Repeat bildet, linearisiert wird durch Einschluß einer Restriktionsendonuklease-Stelle in dem invertierten Tandem-Repeat und Schneiden des Stamms des Stamm-Schlaufe Intermediates mit einer Restriktionsendonuklease.

53. Verfahren nach Anspruch 52, weiterhin umfassend den Schritt des Einführens eines Gens, das eine Restriktionsendonuklease kodiert oder eines Gens, das ein Reverse Transkriptase/RNAse H Polyprotein kodiert, das über einen Prolin-reichen Linker an eine Restriktionsendonuklease in der Zielzelle gebunden ist.

54. Verfahren nach einem der Ansprüche 48 bis 53 zusätzlich das induzierte Fördern der Transkription des Reverse Transkriptase Gens umfassend.

55. Verfahren nach Anspruch 48, umfassend die Schritte des Einführens des Set von genetischen Elementen nach Anspruch 27 in eine Zielzelle, der Transkription des Sets von genetischen Elementen in mRNA, der reversen Transkription des mRNA Transkripts der besagten Kassette mit der Reversen Transkriptase im Zellkern von humanen Gewebekulturzellen, des Verdaus der resultierenden Heteroduplex mit RNAse H und die Entfernung der flankierenden Sequenzen entweder durch (a) Verdau des Stamm-Schlaufe-Intermediats mit Restriktionsendonuklease oder (b) durch vorzeitige Termination des cDNA Transkripts durch Bildung einer Stamm-Schlaufe Sekundärstruktur durch die selbst-komplementären invertierten Tandem Repeats.

56. Pharmazeutische Zusammensetzung, die ein Nukleinsäurekonstrukt oder ein Set von genetischen Elementen nach einem der Ansprüche 1-32, einen Vektor oder ein Vektorsystem nach einem der Ansprüche 38 bis 45 oder eine Wirtszelle nach Anspruch 46 oder 47, zusammen mit einem pharmakologisch akzeptablen Adjuvans, einem Verdünnungsmittel oder einem Träger umfasst

57. Nukleinsäurekonstrukt oder Set von genetischen Elementen nach einem der Ansprüche 1 bis 32, Vektor oder Vektorsystem nach einem der Ansprüche 38 bis 45 oder Wirtszelle nach Anspruch 46 oder 47 zur Verwendung in der Therapie, insbesondere zur Verwendung zur Abgabe eines Inhibitor-Nukleinsäuremoleküls in eine Zielzelle.

58. Nukleinsäurekonstrukt oder Set von genetischen Elementen nach Anspruch 57, wobei das Nukleinsäurekonstrukt oder das Set von genetischen Elementen zur Verwendung in einem *in vivo* Verfahren zur Herstellung eines einzelsträngigen Nukleinsäuremoleküls ist, das eine Sequenz von Interesse aufweist, wobei das Verfahren die Schritte des Einführens eines Nukleinsäurekonstrukts oder eines Set von genetischen Elementen in eine Zielzelle, die Transkription des Nukleinsäurekonstrukts in mRNA und die reverse Transkription des mRNA Transkripts in cDNA umfasst.

59. Nukleinsäurekonstrukt oder Set von genetischen Elementen nach Anspruch 58, wobei die reverse Transkription durch eine Reverse Transkriptase ausgeführt wird, die endogen zu der Zielzelle ist.

60. Nukleinsäurekonstrukt oder Set von genetischen Elementen nach Anspruch 58, wobei das Verfahren weiterhin den Schritt des Einführens eines Gens umfasst, das eine Reverse Transkriptase oder ein Reverse Transkriptase/RNAse H Polyprotein kodiert.

61. Nukleinsäurekonstrukt oder Set von genetischen Elementen nach einem der Ansprüche 58 bis 60, wobei die Herstellung weiterhin den Schritt der Linearisierung des cDNA Transkripts umfasst durch Schneiden der cDNA Stamm-Schlaufe Struktur, die von dem invertierten Tandem-Repeat geformt wird, in dem die Schlaufenstruktur den Stamm verbindet.

62. Nukleinsäurekonstrukt oder Set von genetischen Elementen nach Anspruch 61, wobei die cDNA ,die ein Stamm-Schlaufe-Intermediat durch Watson-Crick-Basenpaarung des invertierten Tandem Repeat bildet, linearisiert wird durch Einschluß einer Restriktionsendonuklease-Stelle in dem invertierten Tandem Repeat, und durch Schneiden des Stamms des Stamm-Schlaufe-Intermediates mit der Restriktionsendonuklease.

63. Nukleinsäurekonstrukt oder Set von genetischen Elementen nach Anspruch 62, wobei das Verfahren weiterhin den Schritt des Einführens eines Gens umfasst, das eine Restriktionsendonuklease kodiert oder eines Gens, das ein Reverse Transkriptase/RNAse H Polyprotein kodiert, das über einen Prolin-reichen Linker an eine Restriktionsendonuklease in der Zielzelle gebunden ist.

64. Nukleinsäurekonstrukt oder Set von genetischen Elementen nach einem der Ansprüche 58 bis 63, wobei das Verfahren zusätzlich das induzierte Fördern der Transkription des Reverse Transkriptase Gens umfasst.

65. Verwendung eines Nukleinsäurekonstrukts oder eines Set von genetischen Elementen nach einem der Ansprüche 1 bis 32, eines Vektors oder eines Vektorsystems nach einem der Ansprüche 38 bis 45 oder einer Wirtszelle nach Anspruch 46 oder 47, zur Herstellung eines Medikaments zur Linderung von krankhaften Zuständen durch Regulierung der Genexpression, insbesondere zur Linderung von krankhaften Zuständen durch Abgabe eines inhibitorischen Nukleinsäuremoleküls in eine Zielzelle.

## Revendications

1. Construction d'acide nucléique pour une administration dans une cellule qui comprend :
des séquences complémentaires 3' et 5' comprenant une unité de répétition en tandem inverse,
une séquence encodant un site de liaison des amorces pour une transcriptase inverse qui est complémentaire à un ARN de transfert (ARNt) et située en position 3' par rapport à l'unité de répétition en tandem inverse, et
une séquence codant pour une séquence d'intérêt située soit entre les séquences complémentaires 3' et 5' de l'unité de répétition en tandem inverse soit entre l'unité de répétition en tandem inverse et la séquence encodant le site de liaison des amorces 3'.

2. Construction d'acide nucléique selon la Revendication 1, dans laquelle la séquence encodant la séquence d'intérêt est située entre les séquences complémentaires 3' et 5' de l'unité de répétition en tandem inverse.

3. Construction d'acide nucléique selon la Revendication 2 qui comprend une deuxième séquence codant pour une séquence d'intérêt située entre l'unité de répétition en tandem inverse et la séquence encodant le site de liaison des amorces 3'.

4. Construction d'acide nucléique selon la Revendication 1, dans laquelle la séquence encodant la séquence d'intérêt est située entre l'unité de répétition en tandem inverse et la séquence encodant le site de liaison des amorces 3'.

5. Construction d'acide nucléique selon l'une quelconque des Revendications 2 à 4, dans laquelle l'unité de répétition en tandem inverse est capable de former un intermédiaire tige-boucle dans un produit d'acide nucléique monocaténaire encodé par ladite construction d'acide nucléique, avec ladite unité de répétition en tandem inverse formant la tige dudit intermédiaire tige-boucle.

6. Construction d'acide nucléique selon l'une quelconque des Revendications 2 à 5, dans laquelle l'unité de répétition en tandem inverse comprend une séquence encodant une ou plusieurs séquence(s) spécifique(s) de reconnaissance enzymatique.

7. Construction d'acide nucléique selon la Revendication 6, dans laquelle la séquence spécifique de reconnaissance enzymatique comprend un site d'endonucléase de restriction.

8. Construction d'acide nucléique selon la Revendication 7, comprenant en outre un gène encodant une endonucléase de restriction.

9. Construction d'acide nucléique selon la Revendication 8, dans laquelle le gène de l'endonucléase de restriction est situé en position 5' par rapport à l'unité de répétition en tandem inverse.

10. Construction d'acide nucléique selon l'une quelconque des Revendications 6 à 9, dans laquelle la séquence spécifique de reconnaissance enzymatique comprend un site de restriction Hind III ou Not I, ou une séquence de reconnaissance pour une endonucléase de restriction sélectionnée dans le groupe constitué de l'endonucléase de type I, l'endonucléase de type II et l'endonucléase de type III.

11. Construction d'acide nucléique selon l'une quelconque des Revendications 1 à 10, dans laquelle l'unité de répétition en tandem inverse comprend une séquence encodant un ou plusieurs site(s) de liaison à l'ADN de protéine eucaryote, procaryote et/ou virale.

12. Construction d'acide nucléique selon l'une quelconque des Revendications 1 à 11, dans laquelle l'unité de répétition en tandem inverse agit suivant une orientation *cis.*

13. Construction d'acide nucléique selon l'une quelconque des Revendications 1 à 12, dans laquelle le site de liaison des amorces est spécifique pour une transcriptase inverse endogène.

14. Construction d'acide nucléique selon l'une quelconque des Revendications 1 à 12, comprenant en outre un gène encodant une transcriptase inverse ou une polyprotéine transcriptase inverse/RNAse H.

15. Construction d'acide nucléique selon la Revendication 14, dans laquelle le gène encodant la transcriptase inverse ou la polyprotéine transcriptase inverse/RNAse H est situé en position 5' par rapport à l'unité de répétition en tandem inverse.

16. Construction d'acide nucléique selon la Revendication 14 ou 15, dans laquelle le gène encodant la polyprotéine transcriptase inverse/RNase H provient du virus de la leucémie murine Moloney, du virus de l'immunodéficience humaine ou du virus de l'immunodéficience simienne.

17. Construction d'acide nucléique selon l'une quelconque des Revendications 14 à 16, dans laquelle le site de liaison des amorces est spécifique pour une transcriptase inverse encodée par le gène de la transcriptase inverse ou de la polyprotéine transcriptase inverse/ RNAse H.

18. Construction d'acide nucléique selon l'une quelconque des revendications précédentes, comprenant en outre un promoteur pour chacune desdites premières ou deuxièmes séquences encodant une séquence d'intérêt, ledit gène de l'endonucléase de restriction, de la transcriptase inverse ou de la transcriptase inverse/RNAse H.

19. Construction d'acide nucléique selon la Revendication 18, dans laquelle le promoteur et/ou l'amplificateur est un promoteur eucaryote.

20. Construction d'acide nucléique selon la Revendication 18 ou 19, dans laquelle le promoteur est un promoteur constitutif, inductible, à large spectre ou spécifique au tissu.

21. Construction d'acide nucléique selon l'une quelconque des revendications précédentes, comprenant en outre une séquence qui encode une séquence de queue de polyadénylation située en position 3' par rapport au site de liaison des amorces 3'.

22. Construction d'acide nucléique selon l'une quelconque des revendications précédentes, dans laquelle la première ou deuxième séquence encodant une séquence d'intérêt comprend une séquence qui encode un ADNsb ayant une activité enzymatique.

23. Construction d'acide nucléique selon la Revendication 22, dans laquelle la première ou deuxième séquence encodant une séquence d'intérêt comprend la séquence 5'-GGCTAGCTACAACGA-3', flanquée dans les directions 5' et 3' par des séquences encodant une ou plusieurs séquence(s) complémentaires à une espèce d'ARNm cible.

24. Construction d'acide nucléique selon la Revendication 23, dans laquelle l'espèce d'ARNm cible concerne :
(i) le gène h-ras,
(ii) l'enzyme c-raf kinase,
(iii) le facteur de croissance angiogénique pléiotrophine, ou
(iv) la région tat du virus de l'immunodéficience simienne (VIS).

25. Ensemble d'éléments génétiques adapté pour être incorporé dans un vecteur pour une administration à une cellule comprenant :
(i) une cassette comprenant une construction d'acide nucléique selon l'une quelconque des Revendications 2 à 4 ; et
(ii) un gène encodant une transcriptase inverse.

26. Ensemble d'éléments génétiques selon la Revendication 25 dans lequel ledit gène de la transcriptase inverse est sélectionné dans le groupe constitué des gènes de la transcriptase inverse provenant du virus de la leucémie murine Moloney ou du virus de l'immunodéficience humaine.

27. Ensemble d'éléments génétiques selon la Revendication 25 comprenant (a) une séquence codant pour une séquence d'intérêt flanquée par des séquences complémentaires 3' et 5' comprenant une unité de répétition en tandem inverse, (b) une séquence encodant un site de liaison des amorces (PBS) pour une transcriptase inverse en position 3' par rapport à l'unité de répétition en tandem inverse, moyennant quoi le PBS est constitué d'une séquence d'acide nucléique complémentaire à une séquence d'ARN de transfert (ARNt) qui réside dans une cellule cible eucaryote, et (c) un gène encodant une transcriptase inverse/RNAse H et une endonucléase de restriction.

28. Construction d'acide nucléique selon l'une quelconque des Revendications 1 à 24, ou ensemble d'éléments génétiques selon l'une quelconque des Revendications 25 à 27, dans lequel la séquence d'intérêt est une molécule d'acide nucléique monocaténaire.

29. Construction d'acide nucléique ou ensemble d'éléments génétiques selon la Revendication 28, dans lequel la molécule d'acide nucléique monocaténaire est l'ADNc ou un ARNm.

30. Construction d'acide nucléique ou ensemble d'éléments génétiques selon l'une quelconque des Revendications 28 ou 29, dans lequel la molécule d'acide nucléique monocaténaire est une molécule d'acide nucléique inhibitrice.

31. Construction d'acide nucléique ou ensemble d'éléments génétiques selon la Revendication 30, dans lequel la molécule d'acide nucléique inhibitrice est une séquence antisens ou un aptamère.

32. Construction d'acide nucléique ou ensemble d'éléments génétiques selon l'une quelconque des Revendications 1 à 31, dans lequel l'acide nucléique est l'ADN.

33. Produit de transcription ARNm comprenant (a) des séquences complémentaires 3' et 5' comprenant une unité de répétition en tandem inverse, (b) un site de liaison des amorces qui est complémentaire à un ARN de transfert (ARNt) et situé en position 3' par rapport à l'unité de répétition en tandem inverse, et (c) une séquence codant pour une séquence d'intérêt située soit entre les séquences complémentaires 3' et 5' de l'unité de répétition en tandem inverse soit entre l'unité de répétition en tandem inverse et le site de liaison des amorces 3'.

34. Produit de transcription ARNm selon la Revendication 33, dans lequel la séquence codant pour la séquence d'intérêt est située entre l'unité de répétition en tandem inverse et le site de liaison des amorces 3'.

35. Produit de transcription ARNm selon l'une quelconque des Revendications 33 ou 34, dans lequel la séquence encodant la séquence d'intérêt comprend une séquence encodant un ADNsb ayant une activité enzymatique.

36. ARNm selon la Revendication 33, qui est un produit de transcription de la construction d'acide nucléique selon la Revendication 32.

37. Produit de transcription ADNsb de l'ARNm selon l'une quelconque des Revendications 33 à 36.

38. Vecteur qui comprend :
des séquences complémentaires 3' et 5' comprenant une unité de répétition en tandem inverse,
une séquence encodant un site de liaison des amorces pour une transcriptase inverse qui est complémentaire à un ARN de transfert (ARNt) et située en position 3' par rapport à l'unité de répétition en tandem inverse, et
un site d'insertion pour une séquence codant pour une séquence d'intérêt entre les séquences complémentaires 3' et 5' de l'unité de répétition en tandem inverse, ou entre l'unité de répétition en tandem inverse et la séquence encodant le site de liaison des amorces 3'.

39. Vecteur selon la Revendication 38, qui comprend un premier site d'insertion entre les séquences complémentaires 3' et 5' de l'unité de répétition en tandem inverse et un deuxième site d'insertion entre l'unité de répétition en tandem inverse et la séquence encodant le site de liaison des amorces 3'.

40. Vecteur selon la Revendication 38 ou 39, comprenant en outre un gène encodant une transcriptase inverse ou une polyprotéine transcriptase inverse/RNAse H.

41. Vecteur selon la Revendication 40, dans lequel le gène de la transcriptase inverse ou de la polyprotéine transcriptase inverse/RNAse H est situé en position 5' par rapport à l'unité de répétition en tandem inverse.

42. Vecteur selon la Revendication 38 comprenant la construction d'acide nucléique ou l'ensemble d'éléments génétiques selon l'une quelconque des Revendications 1 à 32.

43. Système de vecteurs qui comprend un premier vecteur selon la Revendication 38 ou 39, et un deuxième vecteur comprenant un gène qui encode une transcriptase inverse.

44. Vecteur ou système de vecteurs selon l'une quelconque des Revendications 38 à 43, dans lequel le vecteur est un plasmide ou une construction virale modifiée.

45. Vecteur ou système de vecteurs selon l'une quelconque des Revendications 38 à 44, dans lequel le gène est opérationnellement lié à une séquence de contrôle de l'expression.

46. Cellule hôte stablement transformée ou transfectée avec un vecteur ou un système de vecteurs selon l'une quelconque des Revendications 38 à 45 ou comprenant un produit de transcription de l'une quelconque des Revendications 33 à 37.

47. Cellule hôte selon la Revendication 46 qui est une cellule eucaryote ou bactérienne.

48. Procédé de production *in vitro* d'une molécule d'acide nucléique monocaténaire ayant une séquence d'intérêt, lequel procédé comprend les étapes d'introduction d'une construction d'acide nucléique ou d'un ensemble d'éléments génétiques selon l'une quelconque des Revendications 1 à 32 dans une cellule cible, de transcription de la construction d'acide nucléique ou de l'ensemble d'éléments génétiques en ARNm, et de transcription inverse du produit de transcription ARNm en ADNc.

49. Procédé selon la Revendication 48, dans lequel la transcription inverse est effectuée par une transcriptase inverse qui est endogène par rapport à la cellule cible.

50. Procédé selon la Revendication 48, comprenant en outre l'étape d'introduction d'un gène encodant une transcriptase inverse ou une polyprotéine transcriptase inverse/RNAse H dans la cellule cible.

51. Procédé selon l'une quelconque des Revendications 48 à 50, comprenant en outre l'étape de linéarisation du produit de transcription ADNc en coupant la structure tige-boucle de l'ADNc formée par l'unité de répétition en tandem inverse où la structure boucle rejoint la tige.

52. Procédé selon la Revendication 51 dans lequel l'ADNc est linéarisé en incluant un site d'endonucléase de restriction dans l'unité de répétition en tandem inverse, l'ADNc formant un intermédiaire tige-boucle par appariement des bases Watson-Crick de l'unité de répétition en tandem inverse, et en coupant la tige de l'intermédiaire tige-boucle avec une endonucléase de restriction.

53. Procédé selon la Revendication 52, comprenant en outre l'étape d'introduction d'un gène encodant une endonucléase de restriction ou d'un gène encodant une polyprotéine transcriptase inverse/RNAse H liée par un lieur riche en proline à une endonucléase de restriction dans la cellule cible.

54. Procédé selon l'une quelconque des Revendications 48 à 53 comprenant en plus la promotion inductible de la transcription du gène de la transcriptase inverse.

55. Procédé selon la Revendication 48, comprenant les étapes d'introduction de l'ensemble d'éléments génétiques de la Revendication 27 dans une cellule cible, de transcription de l'ensemble d'éléments génétiques en ARNm, de transcription inverse du produit de transcription ARNm de ladite cassette avec la transcriptase inverse dans le noyau des cellules de culture tissulaire humaines, de digestion de l'hétéroduplex résultant avec la RNAse H et d'élimination des séquences flanquantes par (a) digestion de l'endonucléase de restriction de l'intermédiaire tige-boucle ou (b) achèvement prématuré du produit de transcription ADNc par formation d'une structure tige-boucle secondaire par les unités de répétition en tandem inverses autocomplémentaires.

56. Composition pharmaceutique qui comprend une construction d'acide nucléique ou un ensemble d'éléments génétiques selon l'une quelconque des Revendications 1 à 32, un vecteur ou un système de vecteurs selon l'une quelconque des Revendications 38 à 45, ou une cellule hôte selon la Revendication 46 ou 47, conjointement avec un adjuvant, diluant ou véhicule pharmaceutiquement acceptable.

57. Construction d'acide nucléique ou ensemble d'éléments génétiques selon l'une quelconque des Revendications 1 à 32, vecteur ou système de vecteurs selon l'une quelconque des Revendications 38 à 45, ou cellule hôte selon la Revendication 46 ou 47, pour une utilisation en thérapie, en particulier pour une utilisation dans l'administration d'une molécule d'acide nucléique inhibitrice à une cellule cible.

58. Construction d'acide nucléique ou ensemble d'éléments génétiques selon la Revendication 57, dans lequel la construction d'acide nucléique ou l'ensemble d'éléments génétiques est destiné à une utilisation dans un procédé de production *in vivo* d'une molécule d'acide nucléique monocaténaire ayant une séquence d'intérêt, lequel procédé comprend les étapes d'introduction de la construction d'acide nucléique ou de l'ensemble d'éléments génétiques dans une cellule cible, de transcription de la construction d'acide nucléique en ARNm, et de transcription inverse du produit de transcription ARNm en ADNc.

59. Construction d'acide nucléique ou ensemble d'éléments génétiques selon la Revendication 58, dans lequel la transcription inverse est effectuée par une transcriptase inverse qui est endogène par rapport à la cellule cible.

60. Construction d'acide nucléique ou ensemble d'éléments génétiques selon la Revendication 58, dans lequel le procédé comprend en outre l'étape d'introduction d'un gène encodant une transcriptase inverse ou une polyprotéine transcriptase inverse/RNAse H dans la cellule cible.

61. Construction d'acide nucléique ou ensemble d'éléments génétiques selon l'une quelconque des Revendications 58 à 60, dans lequel le procédé comprend en outre l'étape de linéarisation du produit de transcription ADNc en coupant la structure tige-boucle de l'ADNc formée par l'unité de répétition en tandem inverse où la structure boucle rejoint la tige.

62. Construction d'acide nucléique ou ensemble d'éléments génétiques selon la Revendication 61, dans lequel l'ADNc est linéarisé en incluant un site d'endonucléase de restriction dans l'unité de répétition en tandem inverse, l'ADNc formant un intermédiaire tige-boucle par appariement des bases Watson-Crick de l'unité de répétition en tandem inverse, et en coupant la tige de l'intermédiaire tige-boucle avec l'endonucléase de restriction.

63. Construction d'acide nucléique ou ensemble d'éléments génétiques selon la Revendication 62, dans lequel le procédé comprend en outre l'étape d'introduction d'un gène encodant une endonucléase de restriction ou d'un gène encodant une polyprotéine transcriptase inverse/RNAse H liée par un lieur riche en proline à une endonucléase de restriction dans la cellule cible.

64. Construction d'acide nucléique ou ensemble d'éléments génétiques selon l'une quelconque des Revendications 58 à 63, dans lequel le procédé comprend en plus la promotion inductible de la transcription du gène de la transcriptase inverse.

65. Utilisation d'une construction d'acide nucléique ou d'un ensemble d'éléments génétiques selon l'une quelconque des Revendications 1 à 32, d'un vecteur ou d'un système de vecteurs selon l'une quelconque des Revendications 38 à 45, ou d'une cellule hôte selon la Revendication 46 ou 47, pour la fabrication d'un médicament pour soulager une condition pathologique en régulant l'expression génétique, en particulier pour soulager une condition pathologique en administrant une molécule d'acide nucléique inhibitrice à une cellule cible.
